(19) Europäisches Patentamt
European Patent Office
Office européen des brevets

(11) Numéro de publication: 0 367 678 A1

(12) **DEMANDE DE BREVET EUROPEEN**

(21) Numéro de dépôt: 89403029.5

(22) Date de dépôt: 03.11.89

(51) Int. Cl.5: C07D 265/10 , C07D 279/06 , C07D 413/04 , C07D 413/12 , C07D 417/04 , A61K 31/535 , A61K 31/54

Revendications pour les Etats contractants suivants: ES + GR.

(30) Priorité: 03.11.88 FR 8814322

(43) Date de publication de la demande: 09.05.90 Bulletin 90/19

(84) Etats contractants désignés: AT BE CH DE ES FR GB GR IT LI LU NL SE

(71) Demandeur: RHONE-POULENC SANTE
20, avenue Raymond Aron
F-92160 Antony(FR)

(72) Inventeur: Bourzat, Jean-Dominique
20 Boulevard de la Liberation
F-94300 Vincennes(FR)
Inventeur: Cotrel, Claude
17 A Avenue du Docteur Arnold Netter
F-75012 Paris(FR)
Inventeur: Guyon, Claude
17 Bis Avenue Henri Martin
F-94100 Saint Maur des Fosses(FR)
Inventeur: Pitchen, Philippe
"Meadside" Highland Avenue
Brentwood Essex CM15 9DD(FR)

(74) Mandataire: Savina, Jacques et al
RHONE-POULENC SANTE, Service Brevets,
20 Avenue Raymond Aron
F-92165 Antony Cédex(FR)

(54) **Dérives de dihydro-5,6 4H-oxa (ou thia) zine-1,3, leurs procédés de préparation et les médicaments les contenant.**

(57) Composé de formule :

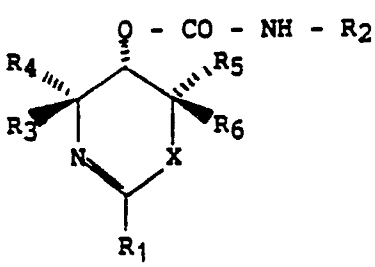

(I)

dans laquelle
- $R_1$ représente un radical indolyl-2, thiényl-2, furyl-3, naphtyle, phényle ou phényle substitué par un ou deux atomes d'halogène, par un radical alcoxy, alkyle, nitro, acylamino, alkylthio, acyle, trifluorométhoxy, morpholino, pipéridino, amino, mono ou dialkylamino, ou en positions -3 et -4 par un radical méthylène-dioxy,
- $R_2$ représente un radical phényle ou phényle substitué par un ou deux atomes d'halogène, par un ou deux radicaux alkyle, par un radical alcoxy, nitro, trifluorométhyle hydroxy ou en positions -3 et -4 par un radical

EP 0 367 678 A1

méthylènedioxy et
- soit X représente un atome d'oxygène et
. $R_3$ représente un radical phényle, $R_4$, $R_5$ et $R_6$ représentent un atome d'hydrogène ou bien
. $R_3$ et $R_4$ représentent un atome d'hydrogène, $R_5$ représente un atome d'hydrogène ou un radical méthyle et $R_6$ représente un radical phényle ou bien
. un des substituants $R_3$ ou $R_4$ représente un radical méthyle et l'autre un atome d'hydrogène, $R_5$ représente un atome d'hydrogène et $R_6$ représente un radical phényle
- soit X représente un atome de soufre, $R_3$ représente un radical phényle et $R_4$, $R_5$ et $R_6$ représentent un atome d'hydrogène ;
étant entendu que les radicaux acyle, alkyle et alcoxy et les portions acyle, alkyle et alcoxy contiennent 1 à 4 atomes de carbone en chaîne droite ou ramifiée ;
leurs sels, leurs procédés de préparation et les médicaments les contenant.

# DERIVES DE DIHYDRO-5,6 4H-OXA (OU THIA) ZINE-1,3, LEURS PROCEDES DE PREPARATION ET LES MEDICAMENTS LES CONTENANT

La présente invention concerne des composés de formule :

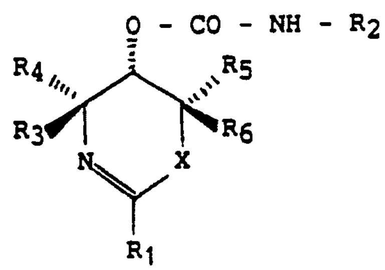

(I)

leurs sels, leurs procédés de préparation et les médicaments les contenant.

Dans la formule (I),

- $R_1$ représente un radical indolyl-2, thiényl-2, furyl-3, naphtyle, phényle ou phényle substitué par un ou deux atomes d'halogène, par un radical alcoxy, alkyle, nitro, acylamino, alkythio, acyle, trifluorométhoxy, morpholino, pipéridino, amino, mono ou dialkylamino dans lesquels les groupes alkyle sont identiques ou différents, ou en positions -3 et -4 par un radical méthylènedioxy,
- $R_2$ représente un radical phényle ou phényle substitué par un ou deux atomes d'halogène, par un ou deux radicaux alkyle, par un radical alcoxy, nitro, trifluorométhyle, hydroxy ou en positions -3 et -4 par un radical méthylènedioxy et
- soit X représente un atome d'oxygène et

. $R_3$ représente un radical phényle, $R_4$, $R_5$ et $R_6$ représentent un atome d'hydrogène ou bien

. $R_3$ et $R_4$ représentent un atome d'hydrogène, $R_5$ représente un atome d'hydrogène ou un radical méthyle et $R_6$ représente un radical phényle ou bien

. un des substituants $R_3$ ou $R_4$ représente un radical méthyle et l'autre un atome d'hydrogène, $R_5$ représente un atome d'hydrogène et $R_6$ représente un radical phényle

- soit X représente un atome de soufre, $R_3$ représente un radical phényle et $R_4$, $R_5$ et $R_6$ représentent un atome d'hydrogène ;

étant entendu que, sauf mention contraire, dans les définitions qui précèdent et celles qui seront citées ci-après, les radicaux alkyle, alcoxy et acyle et les portions alkyle, alcoxy et acyle contiennent 1 à 4 atomes de carbone en chaîne droite ou ramifiée.

L'invention concerne également les sels des composés de formule (I) avec les acides minéraux ou organiques.

Dans la formule (I), les atomes d'halogène sont, de préférence, les atomes de chlore et de fluor.

Selon l'invention, les composés de formule (I) à l'exception de ceux pour lesquels $R_2$ représente un radical phényle substitué par un radical hydroxy peuvent être préparés par action d'un isocyanate de formule :

$R_2$ - NCO (II)

dans laquelle $R_2$ a les mêmes significations que dans la formule (I), à l'exception de représenter un radical phényle substitué par un radical hydroxy sur un composé de formule :

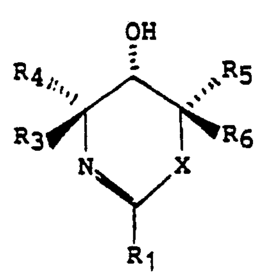

(III)

dans laquelle X, $R_1$, $R_3$, $R_4$, $R_5$ et $R_6$ ont les mêmes significations que dans la formule (I), ou, lorsque l'un

des substituants $R_3$ ou $R_4$ représente un radical méthyle et l'autre un atome d'hydrogène, un mélange de ces composés.

Cette réaction s'effectue, de préférence, dans un solvant inerte tel que le tétrahydrofuranne, le chloroforme, le chlorure de méthylène ou le dichloro-1,2 éthane, à une température comprise entre 20° C et la température d'ébullition du solvant, éventuellement en présence d'une base telle qu'un hydrure de métal alcalin ou la diméthylamino-4 pyridine.

Les isocyanates de formule (II) sont commercialisés ou peuvent être obtenus par application ou adaptation des méthodes décrites par RICHTER et ULRICH ou DROBNICA, KRISTIAN et AUGUSTIN, Patai, "The chemisty of cyanates and their derivatives" Wiley, New York, p. 619-678 et 1003-1221, 1977.

Les composés de formule (III) pour lesquels $R_3$ représente un radical phényle, $R_4$, $R_5$ et $R_6$ représentent un atome d'hydrogène, X représente un atome d'oxygène ou de soufre et $R_1$ a les mêmes significations que dans la formule (I) peuvent être obtenus par cyclisation d'un dérivé de formule :

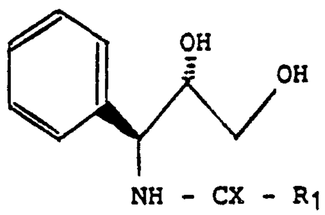

(IV)

dans laquelle $R_1$ et X ont les mêmes significations que précédemment.

Pour les composés pour lesquels X représente un atome d'oxygène, cette réaction s'effectue, généralement, au moyen de chlorure de tosyle, de benzènesulfochlorure ou de chlorure de mésyle, soit en présence d'une amine tertiaire telle que la triéthylamine, dans un solvant chloré tel que chloroforme, le chlorure de méthylène ou le dichloro-1,2 éthane, à une température comprise entre 20° C et 70° C, soit dans la pyridine, à une température comprise entre 20° C et 50° C.

Pour les composés pour lesquels X représente un atome de soufre, cette réaction s'effectue, généralement, dans un solvant inerte tel que le tétrahydrofuranne, en présence de triphénylphosphine et d'azodicarboxylate d'éthyle, à une température voisine de 20° C.

Le dérivé de formule (IV) pour lequel X représente un atome d'oxygène et $R_1$ représente un radical phényle peut être préparé par la méthode décrite par T. SUAMI et al, Bull. Chem. Soc. Japan, 29, 417, 1956.

Les autres dérivés de formule (IV) peuvent être obtenus par action d'un dérivé de formule :

$R_1$ - CX - $R_7$ (V)

dans laquelle X et $R_1$ ont les mêmes significations que précédemment et $R_7$ représente un atome de chlore, on radical alcoxy, alcoxycarbonyloxy ou N-imidazolyle, sur l'amine de formule :

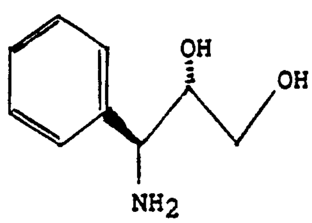

(VI)

Cette réaction s'effectue, de préférence dans un solvant inerte tel que le chloroforme, le chlorure de méthylène, le dichloro-1,2 éthane, l'éther diéthylique, le tétrahydrofuranne, le toluène ou un mélange de ces solvants, à une température comprise entre 20° C et la température d'ébullition du solvant, éventuellement en présence d'une amine tertiaire telle que la triéthylamine.

Les dérivés de formule (V) pour lesquels X représente un atome d'oxygène peuvent être obtenus par action, sur l'acide correspondant, d'un alcool aliphatique saturé tel que le méthanol ou l'éthanol, d'un agent de chloruration tel que dichlorure d'oxalyle ou le chlorure de thionyle, d'un chloroformiate d'alkyle tel que le chloroformiate de méthyle ou d'éthyle ou du carbonyldiimidazole.

L'action de l'alcool aliphatique sur l'acide peut être réalisée au sein dudit alcool comme solvant, en présence d'un acide minéral tel que l'acide sulfurique ou l'acide chlorhydrique, à la température d'ébullition

du milieu réactionnel.

L'action de l'agent de chloruration sur l'acide peut être réalisée dans un solvant inerte tel que l'éther éthylique ou le chloroforme, éventuellement en présence de diméthylformamide, à une température voisine de 20 ° C.

L'action du chloroformiate d'alkyle sur l'acide peut être réalisée dans un solvant inerte tel que le chloroforme ou le chlorure de méthylène, à une température comprise entre -5 ° C et 25 ° C, en présence d'une amine tertiaire telle que la triéthylamine.

L'action du carbonyldiimidazole sur l'acide carboxylique peut être réalisée au sein d'un solvant inerte tel que le tétrahydrofuranne, le diméthylformamide ou le dichlorométhane, à une température voisine de 0 ° C.

Les dérivés de formule (V) pour lesquels X représente un atome de soufre peuvent être préparés par application ou adaptation de la méthode décrite par H. VIOLA et R. MAYER, Z. Chem., vol. 15, 348, 1975, à partir des acides dithiobenzoïques correspondants.

Les acides dithiobenzoïques correspondants peuvent être obtenus par application ou adaptation de la méthode décrite par D.F. AYCOCK et G.R. JURCH jr, J. Org. Chem., 44(4), 569, 1979.

L'amine de formule (VI) peut être préparée selon la méthode décrite par M.F. SAETONE et al, Il Farmaco, 31(3), 209, 1966.

Les composés de formule (III) pour lesquels

- $R_1$ représente un radical indolyl-2, thiényl-2, furyl-3, naphtyle, phényle ou phényle substitué par un ou deux atomes d'halogène, par un radical alcoxy, alkyle, nitro, acyle, trifluorométhoxy ou en positions -3 et -4 par un radical méthylènedioxy,
- X représente un atome d'oxygène et
- soit $R_3$ et $R_4$ représentent un atome d'hydrogène, $R_5$ représente un atome d'hydrogène ou un radical méthyle et $R_6$ représente un radical phényle,
- soit un des substituants $R_3$ ou $R_4$ représente un radical méthyle et l'autre un atome d'hydrogène, $R_5$ représente un atome d'hydrogène et $R_6$ représente un radical phényle ou des mélanges de ces derniers composés,

peuvent être préparés par action d'acide m-chloroperbenzoïque sur un composé de formule :

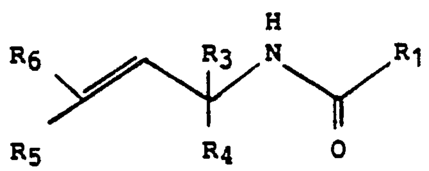 (VII)

dans laquelle $R_1$, $R_3$, $R_4$, $R_5$ et $R_6$ ont les mêmes significations que précédemment puis cyclisation du produit obtenu.

L'action de l'acide m-chloroperbenzoïque sur le composé de formule (VII) s'effectue, généralement, au sein d'un solvant chloré tel que le dichloro-1,2 éthane, le chloroforme ou le chlorure de méthylène, à une température voisine de 0 ° C. La cyclisation s'effectue, de préférence, au moyen d'éthérate de trifluorure de bore, à une température voisine de 20 ° C.

Les composés de formule (VII) peuvent être préparés par application ou adaptation des méthodes décrites par PADWA et al, J. Org. Chem., 44(19), 3281, 1979 et Mc MANUS et al, J. Org. Chem., 43(22), 4288, 1978.

De préférence, on fait réagir un dérivé de formule (V) dans laquelle X représente un atome d'oxygène et $R_1$ et $R_7$ ont les mêmes significations que précédemment sur une amine de formule :

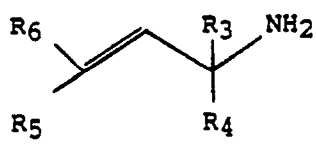 (VIII)

dans laquelle $R_3$, $R_4$, $R_5$ et $R_6$ ont les mêmes significations que dans la formule (VII) .

Cette réaction s'effectue généralement au sein d'un solvant chloré tel que le dichlorométhane ou le chloroforme, en présence d'une amine tertiaire telle que la triéthylamine, à une température comprise entre 20 ° C et 40 ° C.

Les amines de formule (VIII) pour lesquelles l'un des substituants $R_3$ ou $R_4$ représente un radical

méthyle et l'autre un atome d'hydrogène, $R_5$ représente un atome d'hydrogène et $R_6$ représente un radical phényle peuvent être obtenues, par le procédé décrit par T.G. SCHENK et al, J. Am. Chem. Soc., 107(7), 2064, 1985.

L'amine de formule (VIII) pour laquelle $R_3$ et $R_4$ représentent un atome d'hydrogène, $R_5$ représente un radical méthyle et $R_6$ représente un radical phényle peut être obtenue par réduction du nitrile correspondant. Cette réduction s'effectue généralement au moyen d'hydrure de lithium et d'aluminium, de préférence au sein de l'éther diéthylique, à une température de -15° C.

Le nitrile correspondant peut être obtenu par la méthode décrite par TEXIER et al, Synthesis, 884, 1979.

Les composés de formule (III) pour lesquels

- $R_1$ représente un radical phényle substitué par un radical amino, mono ou dialkylamino, acylamino, alkylthio, morpholino ou pipéridino
- X représente un atome d'oxygène et
- soit $R_3$ et $R_4$ représentent un atome d'hydrogène, $R_5$ représente un atome d'hydrogène ou un radical méthyle et $R_5$ représente un radical phényle,
- soit un des substituants $R_3$ ou $R_4$ représente un radical méthyle et l'autre un atome d'hydrogène, $R_5$ représente un atome d'hydrogène et $R_6$ représente un radical phényle ou des mélanges de ces derniers composés,

peuvent être préparés par cyclisation d'un composé de formule :

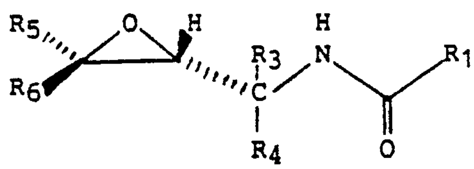 (IX)

dans laquelle $R_1$, $R_3$, $R_4$, $R_5$ et $R_6$ ont les significations mentionnées ci-dessus.

Cette cyclisation s'effectue généralement au moyen d'éthérate de trifluorure de bore, au sein d'un solvant inerte tel que le dichloro-1,2 éthane, le chloroforme, le chlorure de méthylène ou l'éther diéthylique, à une température voisine de 20° C.

Les composés de formule (IX) peuvent être obtenus par hydrolyse d'un amide de formule :

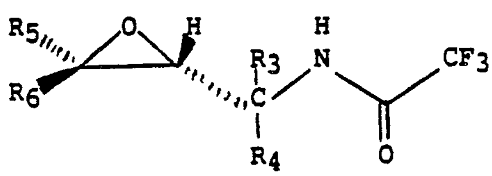 (X)

dans laquelle $R_3$, $R_4$, $R_5$ et $R_6$ ont les mêmes significations que dans la formule (IX), suivie de l'action du produit ainsi obtenu et isolé sous forme d'une solution dans un solvant inerte tel que le dichlorométhane sur un dérivé de formule (V) dans laquelle $R_1$ représente un radical phényle substitué par un radical amino, mono ou dialkylamino, acylamino, alkylthio, morpholino ou pipéridino et $R_7$ représente un radical N-imidazolyle.

L'hydrolyse peut être réalisée au moyen d'un hydroxyde alcalin comme la potasse, en milieu aqueux, à une température voisine de 20° C.

L'action du produit hydrolysé en solution dans un solvant inerte sur un dérivé de formule (V) s'effectue à une température voisine de 20° C.

Les composés de formule (X) peuvent être préparés par action d'acide m-chloroperbenzoïque sur un dérivé de formule :

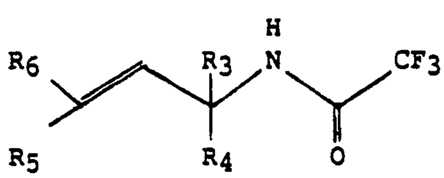 (XI)

dans laquelle $R_3$, $R_4$, $R_5$ et $R_6$ ont les mêmes significations que dans la formule (X).

Cette réaction s'effectue, de préférence, au sein d'un solvant chloré tel que le dichloro-1,2 éthane, le chloroforme ou le chlorure de méthylène, à une température voisine de 0° C.

Les dérivés de formule (XI) peuvent être obtenus par application ou adaptation de la méthode décrite par P. HODGE et al, Synthesis, 941, 1984.

Selon l'invention, les composés de formule (I) peuvent également être préparés par action d'une amine de formule :

$R_2 - NH_2$ (XII)

dans laquelle $R_2$ a les mêmes significations que dans la formule (I) sur un composé de formule :

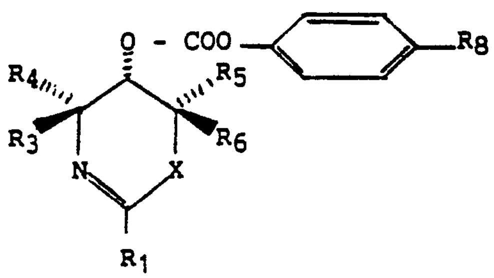

(XIII)

dans laquelle $R_8$ représente un atome d'hydrogène ou un radical nitro et X, $R_1$, $R_3$, $R_4$, $R_5$ et $R_6$ ont les mêmes significations que dans la formule (I) ou, lorsque l'un des substituants $R_3$ ou $R_4$ représente un radical méthyle et l'autre un atome d'hydrogène, un mélange de ces composés.

Cette réaction s'effectue, généralement, dans un solvant organique tel que l'acétonitrile, en présence d'une base telle la diméthylamino-4 pyridine ou la triéthylamine, à une température comprise entre 50° C et la température d'ébullition du solvant.

Les composés de formule (XIII) peuvent être préparés par action du chloroformiate de phényle ou du chloroformiate de nitro-4 phényle sur un composé de formule (III) dans laquelle X, $R_1$, $R_3$, $R_4$, $R_5$ et $R_6$ ont les mêmes significations que dans la formule (I) ou, lorsque l'un des substituants $R_3$ ou $R_4$ représente un radical méthyle et l'autre un atome d'hydrogène, un mélange de ces composés.

Cette réaction s'effectue, généralement, dans la pyridine, à une température voisine de 20° C.

Comme l'homme du métier pourra s'en rendre compte, certains radicaux entrant dans la définition du symbole $R_1$ sont incompatibles avec des réactifs mis en oeuvre au cours des réactions et doivent être protégés préalablement à la mise en oeuvre des procédés ou de certaines phases des procédés exposés précédemment. C'est notamment le cas lorsque le radical $R_1$ contient des fonctions amines primaires ou secondaires. Dans ce cas, lesdites fonctions devront être protégées par toute méthode connue de l'homme du métier puis être débloquées après réaction.

Les mélanges réactionnels obtenus par les divers procédés décrits précédemment peuvent être purifiés par les méthodes classiques physiques ou chimiques de purification (évaporation, extrac tion, cristallisation, chromatographie ...).

Les composés de formule (I), sous forme de base libre, peuvent être transformés en sel d'addition avec les acides par action d'un acide dans un solvant organique tel qu'un alcool, une cétone, un solvant chloré ou un éther.

Les composés de formule (I) présentent des propriétés pharmacologiques intéressantes. Ces composés sont des antagonistes de cholécystokinine (CCK) et sont donc utiles dans le traitement et la prévention des désordres liés à la CCK au niveau du système nerveux et de l'appareil gastrointestinal. C'est ainsi que ces composés peuvent être utilisés pour le traitement ou la prévention des psychoses, de la maladie de Parkinson, de la diskinésie tardive, du syndrome du colon irritable, de la pancréatite aigüe, des ulcères et des désordres de la motilité intestinale et comme régulateur de l'appétit.

Ces composés ont également un effet de potentialisation sur l'activité analgésique des médicaments analgésiques narcotiques et non narcotiques.

L'activité anti-CCK des composés de formule (I) a été déterminée selon une technique inspirée de celle de A. SAITO et al, J. Neuro. Chem., 37, 483-490, 1981. Dans ce test, la $CI_{50}$ des composés de formule (I) est inférieure à 1000 nM.

Les composés de formule (I) présentent une toxicité faible. Leur $DL_{50}$ est généralement supérieure à 40 mg/kg par voie sous-cutanée chez la souris.

D'un intérêt particulier, sont les composés de formule (I) pour lesquels :

- $R_1$ représente un radical thiényl-2, naphtyle ou phényle éventuellement substitué par un ou deux atomes

d'halogène, par un radical alcoxy, alkyle, nitro, acyle, alkylthio, trifluorométhoxy, morpholino, pipéridino, amino, mono ou dialkylamino, ou en positions -3 et -4 par un radical méthylénedioxy,
- $R_2$ représente un radical phényle éventuellement substitué par un ou deux atomes d'halogène, par un radical alcoxy, nitro, alkyle, hydroxy, trifluorométhyle ou en positions -3 et -4 par un radical méthylènedioxy, et
- X représente un atome d'oxygène.

Sont particulièrement intéressants les composés suivants :
- (chloro-4 phényl)-2 phényl-4 phénylcarbamoyloxy-5 dihydro-5,6 4H-oxazine-1,3-(4RS, 5RS)
- (méthylènedioxy-3,4 phényl)-2 phényl-4 phénylcarbamoyloxy-5 dihydro-5,6 4H-oxazine-1,3-(4RS, 5RS)
- (méthoxy-4 phényl)-2 phényl-4 phénylcarbamoyloxy-5 dihydro-5,6 4H-oxazine-1,3-(4RS, 5RS)
- diphényl-2,6 phénylcarbamoyloxy-5 dihydro-5,6 4H-oxazine-1,3-(5RS, 6SR)
- (chloro-3 phénylcarbamoyloxy)-5 diphényl-2,6 dihydro-5,6 4H-oxazine-1,3-(5RS, 6SR)
- (méthyl-3 phénylcarbamoyloxy)-5 diphényl-2,6 dihydro-5,6 4H-oxazine-1,3-(5RS, 6SR)
- (diméthylamino-4 phényl)-2 phényl-6 phénylcarbamoyloxy-5 dihydro-5,6 4H-oxazine-1,3-(5RS, 6SR)
- (méthylamino-4 phényl)-2 phényl-6 phénylcarbamoyloxy-5 dihydro-5,6 4H-oxazine-1,3-(5RS, 6SR)
- (éthylthio-4 phényl)-2 phényl-6 phénylcarbamoyloxy-5 dihydro-5,6 4H-oxazine-1,3-(5RS, 6SR)
- (hydroxy-3 phénylcarbamoyloxy)-5 diphényl-2,6 dihydro-5,6 4H-oxazine-1,3-(5RS, 6SR)
- (méthoxy-4 phényl)-2 phényl-6 phénylcarbamoyloxy-5 dihydro-5,6 4H-oxazine-1,3-(5RS, 6SR)
- (chloro-4 phényl)-2 phényl-6 phénylcarbamoyloxy-5 dihydro-5,6 4H-oxazine-1,3-(5RS, 6SR)
- (fluoro-2 phényl)-2 phényl-6 phénylcarbamoyloxy-5 dihydro-5,6 4H-oxazine-1,3-(5RS, 6SR)
- (dichloro-3,4 phényl)-2 phényl-6 phénylcarbamoyloxy-5 dihydro-5,6 4H-oxazine-1,3-(5RS, 6SR)
- (fluoro-4 phényl)-2 phényl-6 phénylcarbamoyloxy-5 dihydro-5,6 4H-oxazine-1,3-(5RS, 6SR)
- (dichloro-3,4 phénylcarbamoyloxy)-5 diphényl-2,4 dihydro-5,6 4H-oxazine-1,3-(4RS, 5RS)
- phényl-6 phénylcarbamoyloxy-5 (thiényl-2)-2 dihydro-5,6 4H-oxazine-1,3-(5RS, 6SR)
- (méthylènedioxy-3,4 phénylcarbamoyloxy)-5 diphényl-2,4 dihydro-5,6 4H-oxazine-1,3-(4RS, 5RS)
- (méthoxy-3 phénylcarbamoyloxy)-5 diphényl-2,6 dihydro-5,6 4H-oxazine-1,3-(5RS, 6SR)
- (nitro-3 phénylcarbamoyloxy)-5 diphényl-2,6 dihydro-5,6 4H-oxazine-1,3-(5RS, 6SR)
- méthyl-6 diphényl-2,6 phénylcarbamoyloxy-5 dihydro-5,6 4H-oxazine-1,3-(5RS, 6SR)
- (acétyl-4 phényl)-2 phényl-6 phénylcarbomoyloxy-5 dihydro-5,6 4H-oxazine-1,3 (5RS, 6SR)
- (isopropyl-4 phényl)-2 phényl-6 phénylcarbomoyloxy-5 dihydro-5,6 4H-oxazine-1,3 (5RS, 6SR)
- (morpholino-4 phényl)-2 phényl-6 phénylcarbomoyloxy-5 dihydro-5,6 4H-oxazine-1,3 (5RS, 6SR)
- (pipéridino-4 phényl)-2 phényl-6 phénylcarbomoyloxy-5 dihydro-5,6 4H-oxazine-1,3 (5RS, 6SR).

Pour l'emploi thérapeutique, il peut être fait usage des composés de formule (I) tels quels ou à l'état de sels pharmaceutiquement acceptables. Comme exemples de sels peuvent être cités les sels d'addition avec les acides minéraux tels que chlorhydrates, sulfates, nitrates, phosphates, ou organiques tels que acétates, propionates, succinates, benzoates, fumarates, maléates, méthanesulfonates, isothionates, théophillineacétates, salicylates, phénolphtalinates, méthylène-bis-β-oxynaphtoates ou des dérivés de substitution de ces dérivés.

Les exemples suivants, donnés à titre non limitatif, montrent comment l'invention peut être mise en pratique.

## EXEMPLE 1

A une solution de 18 g de diphényl-2,4 dihydro-5,6 4H-oxazine-1,3 ol-5-(4RS, 5RS) dans 200 cm3 de dichloro-1,2 éthane maintenue sous atmosphère d'argon, on ajoute à une température voisine de 20 °C, 9 g d'isocyanate de phényle. La solution obtenue est agitée à reflux pendant 2 heures puis concentrée à sec sous pression réduite (2,7 kPa). Le résidu est purifié par chromatographie sur 400 g de silice (0,063 - 0,2 mm) contenus dans une colonne de 7 cm de diamètre en recueillant des fractions de 50 cm3. Les fractions 1 à 12, éluées par un mélange d'acétate d'éthyle et de cyclohexane (1-6 en volumes), sont réunies et concentrées à sec sous pression réduite (2,7 kPa) à 40 °C. On obtient, après recristallisation dans l'acétonitrile, 7 g de diphényl-2,4 phénylcarbamoyloxy-5 dihydro-5,6 4H-oxazine-1,3-(4RS, 5RS) fondant à 163 °C.

Le diphényl-2,4 dihydro-5,6 4H-oxazine-1,3 ol-5-(4RS, 5RS) peut être préparé de la manière suivante : à une solution de 5,42 g de N-[dihydroxy-2,3 phényl-1 propyl-(1RS, 2RS)] benzamide et de 8,35 cm3 de triéthylamine dans 200 cm3 de dichloro-1,2 éthane maintenue sous atmosphère d'argon, on ajoute à une température voisine de 20 °C, 4,2 g de chlorure de tosyle. La solution obtenue est agitée à une température voisine de 70 °C pendant 4 heures puis ajoutée à 200 cm3 d'eau distillée. La phase organique est lavée par

2 fois 100 cm3 d'eau distillée puis par 100 cm3 de saumure, séchée sur sulfate de magnésium, filtrée puis concentrée à sec sous pression réduite (2,7 kPa) à 40 °C. On obtient, après recristallisation dans l'acétonitrile, 2,6 g de diphényl-2,4 dihydro-5,6 4H-oxazine-1,3 ol-5-(4RS, 5RS) fondant à 150 °C.

Le N-[dihydroxy-2,3 phényl-1 propyl-(1RS, 2RS)] benzamide peut être préparé selon la méthode décrite par T. SUAMI, I. UCHIDA, S. UMEZAWA, Bull. Chem. Soc. Japan, 1956, 29, 417.

EXEMPLE 2

A une solution de 1,52 g de diphényl-2,4 dihydro-5,6 4H-oxazine-1,3 ol-5-(4RS, 5RS) dans 20 cm3 de dichloro-1,2 éthane maintenue sous atmosphère d'argon, on ajoute à une température voisine de 20 °C, 1,15 g d'isocyanate de dichloro-3,4 phényle. La solution obtenue est chauffée à reflux pendant 6 heures puis concentrée à sec sous pression réduite (2,7 kPa). On obtient, après recristallisation dans l'acétonitrile, 2 g de (dichloro-3,4 phénylcarbamoyloxy)-5 diphényl-2,4 dihydro-5,6 4H-oxazine-1,3-(4RS, 5RS) fondant à 184 °C.

EXEMPLE 3

A une solution de 1,7 g de diphényl-2,4 dihydro-5,6 4H-oxazine-1,3 ol-5-(4RS, 5RS) dans 20 cm3 de dichloro-1,2 éthane maintenue sous atmosphère d'argon, on ajoute à une température voisine de 20 °C, 1 g d'isocyanate de méthoxy-4 phényle. La solution obtenue est agitée à reflux pendant 4 heures puis concentrée à sec sous pression réduite (2,7 kPa). Le résidu est purifié par chromatographie sur 40 g de silice (0,063 - 0,2 mm) contenus dans une colonne de 2 cm de diamètre en recueillant des fractions de 20 cm3. Les fractions 2 à 6, éluées par un mélange d'acétate d'éthyle et de cyclohexane (3-7 en volumes), sont réunies et concentrées à sec sous pression réduite (2,7 kPa) à 40 °C. On obtient, après recristallisation dans l'oxyde de diisopropyle, 0,7 g de (méthoxy-4 phénylcarbamoyloxy)-5 diphényl-2,4 dihydro-5,6 4H-oxazine-1,3-(4RS, 5RS) fondant à 140 °C.

EXEMPLE 4

A une solution de 1,5 g de diphényl-2,4 dihydro-5,6 4H-oxazine-1,3 ol-5-(4RS, 5RS) dans 20 cm3 de dichloro-1,2 éthane maintenue sous atmosphère d'argon, on ajoute à une température voisine de 20 °C, 1,08 g d'isocyanate de nitro-4 phényle. La solution obtenue est chauffée à reflux pendant 4 heures puis concentrée à sec sous pression réduite (2,7 kPa). Le résidu est purifié par chromatographie sur 40 g de silice (0,063 - 0,2 mm) contenus dans une colonne de 2 cm de diamètre en recueillant des fractions de 20 cm3. Les fractions 2 à 7, éluées par un mélange d'acétate d'éthyle et de cyclohexane (2-3 en volumes), sont réunies et concentrées à sec sous pression réduite (2,7 kPa) à 40 °C. On obtient, après recristallisation dans un mélange d'acétate d'éthyle et d'oxyde de diisopropyle (1-1 en volumes), 0,8 g de (nitro-4 phénylcarbamoyloxy)-5 diphényl-2,4 dihydro-5,6 4H-oxazine-1,3-(4RS, 5RS) fondant à 220 °C.

EXEMPLE 5

A une solution de 1,2 g de diphényl-2,4 dihydro-5,6 4H-oxazine-1,3 ol-5-(4RS, 5RS) dans 20 cm3 de dichloro-1,2 éthane maintenue sous atmosphère d'argon, on ajoute à une température voisine de 20 °C, 0,8 g d'isocyanate de chloro-3 phényle. La solution obtenue est chauffée à reflux pendant 7 heures et 30 minutes puis concentrée à sec sous pression réduite (2,7 kPa). Le résidu est purifié par chromatographie sur 40 g de silice (0,063 - 0,2 mm) contenus dans une colonne de 2 cm de diamètre en recueillant des fractions de 15 cm3. Les fractions 1 à 3, éluées par un mélange d'acétate d'éthyle et de cyclohexane (3-7 en volumes), sont réunies et concentrées à sec sous pression réduite (2,7 kPa) à 40 °C. On obtient, après recristallisation dans l'acétonitrile, 1,4 g de (chloro-3 phénylcarbamoyloxy)-5 diphényl-2,4 dihydro-5,6 4H-oxazine-1,3-(4RS, 5RS) fondant à 160 °C.

EXEMPLE 6

A une solution de 1,2 g de diphényl-2,4 dihydro-5,6 4H-oxazine-1,3 ol-5-(4RS, 5RS) dans 20 cm3 de dichloro-1,2 éthane maintenue sous atmosphère d'argon, on ajoute à une température voisine de 20° C, 1 g d'isocyanate de chloro-4 phényle. La solution obtenue est chauffée à reflux pendant 7 heures et 30 minutes puis concentrée à sec sous pression réduite (2,7 kPa). On obtient, après 2 recristallisations successives dans l'acétonitrile, 1,1 g de (chloro-4 phénylcarbamoyloxy)-5 diphényl-2,4 dihydro-5,6 4H-oxazine-1,3-(4RS, 5RS) fondant à 200° C.

EXEMPLE 7

A une solution de 1,26 g de diphényl-2,4 dihydro-5,6 4H-oxazine-1,3 ol-5-(4RS, 5RS) dans 30 cm3 de dichloro-1,2 éthane maintenue sous atmosphère d'argon, on ajoute à une température voisine de 20° C, 0,8 g d'isocyanate de trifluorométhyl-4 phényle. La solution obtenue est chauffée à reflux pendant 5 heures puis concentrée à sec sous pression réduite (2,7 kPa). Le résidu est purifié par chromatographie sur 40 g de silice (0,063 - 0,2 mm) contenus dans une colonne de 1 cm de diamètre en recueillant des fractions de 20 cm3. Les fractions 2 et 3, éluées par un mélange d'acétate d'éthyle et de cyclohexane (3-7 en volumes), sont réunies et concentrées à sec sous pression réduite (2,7 kPa) à 40° C. On obtient, après recristallisation dans l'éther de pétrole, 1,4 g de diphényl-2,4 (trifluorométhyl-4 phénylcarbamoyloxy)-5 dihydro-5,6 4H-oxazine-1,3-(4RS, 5RS) fondant à 140° C.

EXEMPLE 8

A une solution de 5,15 g d'(indolyl-2)-2 phényl-4 dihydro-5,6 4H-oxazine-1,3 ol-5-(4RS, 5RS) dans 50 cm3 de dichloro-1,2 éthane maintenue sous atmosphère d'argon, on ajoute à une température voisine de 20° C, 2,3 g d'isocyanate de phényle. La solution obtenue est agitée 7 heures à reflux puis concentrée à sec sous pression réduite (2,7 kPa) à 40° C. On obtient après 2 recristallisations successives dans l'oxyde de diisopropyle, 4 g d'(indolyl-2)-2 phényl-4 phénylcarbamoyloxy-5 dihydro-5,6 4H-oxazine-1,3-(4RS, 5RS) fondant à 166° C.

L'(indolyl-2)-2 phényl-4 dihydro-5,6 4H-oxazine-1,3 ol-5-(4RS, 5RS) peut être préparé de la manière suivante : à une solution de 18,6 g de N-[dihydroxy-2,3 phényl-1 propyl-(1RS, 2RS)] indolecarboxamide-2 dans 200 cm3 de pyridine maintenue sous atmosphère d'argon, on ajoute à une température voisine de 20° C, 13,77 g de chlorure de tosyle. La solution obtenue est agitée pendant 6 heures à une température voisine de 40° C puis 16 heures à une température voisine de 20° C et est ensuite versée dans 1800 cm3 d'eau. On extrait par 3 fois 250 cm3 d'acétate d'éthyle et les phases organiques sont réunies, lavées à l'eau, séchées sur sulfate de magnésium, filtrées et concentrées à sec sous pression réduite (2,7 kPa) à 40° C. Le résidu est purifié par chromatographie sur 500 g de silice (0,063-0,2 mm) contenus dans une colonne de 7 cm de diamètre, en recueillant des fractions de 50 cm3. Les fractions 3 à 10, éluées par un mélange d'acétate d'éthyle et de cyclohexane (1-1 en volumes), sont réunies et concentrées à sec sous pression réduite (2,7 kPa) à 40° C. On obtient, après recristallisation dans l'acétate d'éthyle, 3,2 g d'(indolyl-2)-2 phényl-4 dihydro-5,6 4H-oxazine-1,3 ol-5-(4RS, 5RS) fondant à 204° C.

Le N-[dihydroxy-2,3 phényl-1 propyl-(1RS, 2RS)] indolecarboxamide-2 peut être préparé de la manière suivante ; à une solution de 8,05 g d'acide indolecarboxylique-2 et de 0,1 cm3 de diméthylformamide dans 90 cm3 d'éther diéthylique maintenue sous atmosphère d'argon, on ajoute goutte à goutte à une température voisine de 20° C, 5,15 cm3 de dichlorure d'oxalyle dissous dans 10 cm3 d'éther diéthylique. La solution obtenue est agitée 3 heures à une tempé rature voisine de 20° C, puis ajoutée goutte à goutte à une solution de 7,6 g d'amino-3 phényl-3 propanediol-1,2-(2RS, 3RS) et de 8,45 cm3 de triéthylamine dans 100 cm3 de dichlorométhane, maintenue sous atmosphère d'argon. La suspension est agitée pendant 2 heures à une température voisine de 20° C puis versée dans 200 cm3 d'eau distillée. Le précipité est filtré, lavé à l'eau distillée et séché. On obtient, après 2 recristallisations successives dans l'acétate d'éthyle, 5,8 g de N-[dihydroxy-2,3 phényl-1 propyl-(1RS, 2RS)] indolecarboxamide-2 fondant à 151° C.

L'amino-3 phényl-3 propanediol-1,2-(2RS, 3RS) peut être préparé selon la méthode décrite par M.F. SAETONE, Il farmaco-Ed. Sc., 1966, 31(3), 209.

EXEMPLE 9

A une solution de 1,1 g de (chloro-4 phényl)-2 phényl-4 dihydro-5,6 4H-oxazine-1,3 ol-5-(4RS, 5RS)

dans 20 cm3 de dichloro-1.2 éthane maintenue sous atmosphère d'argon, on ajoute à une température voisine de 20 °C, 0,5 g d'isocyanate de phényle. La solution obtenue est chauffée à 70 °C pendant 2 heures puis concentrée à sec sous pression réduite (2,7 kPa). Le résidu est purifié par chromatographie sur 40 g de silice (0.063 - 0,2 mm) contenus dans une colonne de 2 cm de diamètre en recueillant des fractions de 20 cm3. Les fractions 2 à 5, éluées par un mélange de dichlorométhane et de méthanol (98-2 en volumes), sont réunies et concentrées à sec sous pression réduite (2,7 kPa) à 40 °C. On obtient, après recristallisation dans on mélange d'oxyde de diisopropyle et d'acétate d'éthyle (1-1 en volumes), 1,25 g de (chloro-4 phényl)-2 phényl-4 phénylcarbamoyloxy-5 dihydro-5,6 4H-oxazine-1,3-(4RS, 5RS) fondant à 171 °C.

Le (chloro-4 phényl)-2 phényl-4 dihydro-5,6 4H-oxazine-1,3 ol-5-(4RS, 5RS) peut être préparé de la manière suivante ; à une solution de 3,2 g de N-[dihydroxy-2,3 phényl-1 propyl-(1RS, 2RS)] chloro-4 benzamide et de 4,4 cm3 de triéthylamine dans 80 cm3 de dichloro-1,2 éthane maintenue sous atmosphère d'argon, on ajoute à une température voisine de 60 °C, 2,9 g de chlorure de tosyle. La solution obtenue est chauffée pendant 16 heures à une température voisine de 60 °C puis ajoutée à 50 cm3 d'eau distillée. La phase aqueuse est neutralisée par une solution aqueuse d'acide chlorhydrique 5N puis extraite par 3 fois 50 cm3 de dichlorométhane. Les phases organiques sont réunies, lavées à l'eau, séchées sur sulfate de magnésium, filtrées et concentrées à sec sous pression réduite (2,7 kPa) à 40 °C. Le résidu est purifié par chromatographie sur 100 g de silice (0,063 - 0,2 mm) contenus dans une colonne de 3 cm de diamètre, en recueillant des fractions de 25 cm3. Les fractions 3 à 10, éluées par un mélange d'acétate d'éthyle et de cyclohexane (2-3 en volumes), sont réunies et concentrées à sec sous pression réduite (2,7 kPa) à 40 °C. On obtient après recristallisation dans l'oxyde de diisopropyle, 1,1 g de (chloro-4 phényl)-2 phényl-4 dihydro-5,6 4H-oxazine-1,3 ol-5-(4RS, 5RS) fondant à 142 °C.

Le N-[dihydroxy-2,3 phényl-1 propyl-(1RS, 2RS)] chloro-4 benzamide peut être préparé de la manière suivante : en opérant comme à l'exemple 8 pour la préparation du N-[dihydroxy-2,3 phényl-1 propyl-(1RS, 2RS)] indolecarboxamide-2, mais à partir de 2,57 g d'acide chloro-4 benzoïque, de 1,55 cm3 de dichlorure d'oxalyle, de 2,5 g d'amino-3 phényl-3 propanediol-1,2-(2RS, 3RS) et de 8,3 cm3 de triéthylamine, on obtient, après lavage du précipité à l'eau distillée puis à l'oxyde de diisopropyle, 3,2 g de N-[dihydroxy-2,3 phényl-1 propyl-(1RS, 2RS)] chloro-4 benzamide f.ndant à 142 °C.

## EXEMPLE 10

A une solution de 1,9 g de (méthylènedioxy-3,4 phényl)-2 phényl-4 dihydro-5,6 4H-oxazine-1,3 ol-5-(4RS, 5RS) dans 30 cm3 de dichloro-1,2 éthane maintenue sous atmosphère d'argon, on ajoute à une température voisine de 20 °C, 0,84 g d'isocyanate de phényle. La solution obtenue est chauffée à 80 °C pendant 4 heures puis concentrée à sec sous pression réduite (2,7 kPa). Le résidu est purifié par chromatographie sur 100 g de silice (0,063 - 0,2 mm) contenus dans une colonne de 3 cm de diamètre en recueillant des fractions de 20 cm3. Les fractions 2 à 8, éluées par un mélange de dichlorométhane et de méthanol (98-2 en volumes), sont réunies et concentrées à sec sous pression réduite (2,7 kPa) à 40 °C. On obtient, après recristallisation dans un mélange d'oxyde de diisopropyle et d'acé tate d''éthyle (1-1 en volumes), 1,3 g de (méthylènedioxy-3,4 phényl)-2 phényl-4 phénylcarbamoyloxy-5 dihydro-5,6 4H-oxazine-1,3-(4RS, 5RS) fondant à 115 °C.

Le (méthylènedioxy-3,4 phényl)-2 phényl-4 dihydro-5,6 4H-oxazine-1,3 ol-5-(4RS, 5RS) peut être préparé de la manière suivante : à une solution de 6 g de N-[dihydroxy-2,3 phényl-1 propyl-(1RS, 2RS)] méthylènedioxy-3,4 benzamide et de 8 cm3 de triéthylamine dans 80 cm3 de dichloro-1,2 éthane maintenue sous atmosphère d'argon, on ajoute à une température voisine de 60 °C, 4 g de chlorure de tosyle. La solution obtenue est chauffée pendant 16 heures à une température voisine de 60 °C puis ajoutée à 50 cm3 d'eau distillée. La phase aqueuse est neutralisée par une solution aqueuse d'acide chlorhydrique 5N puis extraite par 3 fois 50 cm3 de dichlorométhane. Les phases organiques sont réunies, lavées à l'eau, séchées sur sulfate de magnésium, filtrées et concentrées à sec sous pression réduite (2,7 kPa) à 40 °C. Le résidu est purifié par chromatographie sur 200 g de silice (0,063 - 0,2 mm) contenus dans une colonne de 3 cm de diamètre, en recueillant des fractions de 25 cm3. Les fractions 5 à 10, éluées par un mélange d'acétate d'éthyle et de cyclohexane (1-1 en volumes), sont réunies et concentrées à sec sous pression réduite (2,7 kPa) à 40 °C. On obtient après recristallisation dans l'oxyde de diisopropyle, 1,9 g de (méthylènedioxy-3,4 phényl)-2 phényl-4 dihydro-5,6 4H-oxazine-1,3 ol-5-(4RS, 5RS) fondant à 137 °C.

Le N-[dihydroxy-2,3 phényl-1 propyl-(1RS, 2RS)] méthylènedioxy-3,4 benzamide peut être préparé de la manière suivante : en opérant comme à l'exemple 8 pour la préparation du N-[dihydroxy-2,3 phényl-1 propyl-(1RS, 2RS)] indolecarboxamide-2, mais à partir de 4,6 g d'acide pipéronylique, de 2,6 cm3 de

dichlorure d'oxalyle, de 4,17 g d'amino-3 phényl-3 propanediol-1,2-(2RS, 3RS) et de 10,5 cm3 de triéthylamine, on obtient après lavage du précipité à l'eau distillée puis à l'oxyde de diisopropyle, 6 g de N-[dihydroxy-2,3 phényl-1 propyl-(1RS, 2RS)] méthylénedioxy-3,4 benzamide fondant à 172° C.

EXEMPLE 11

A une solution de 1,75 g de (méthoxy-4 phényl)-2 phényl-4 dihydro-5,6 4H-oxazine-1,3 ol-5-(4RS, 5RS) dans 20 cm3 de dichloro-1,2 éthane maintenue sous atmosphère d'argon, on ajoute à une température voisine de 20° C, 0,6 g d'isocyanate de phényle. La solution obtenue est chauffée à 60° C pendant 4 heures puis concentrée à sec sous pression réduite (2,7 kPa). Le résidu est purifié par chromatographie sur 100 g de silice (0,063 - 0,2 mm) contenus dans une colonne de 3 cm de diamètre en recueillant des fractions de 20 cm3. Les fractions 2 à 6, éluées par un mélange d'acétate d'éthyle et de cyclohexane (3-7 en volumes), sont réunies et concentrées à sec sous pression réduite (2,7 kPa) à 40° C. On obtient, après recristallisation dans un mélange d'oxyde de diisopropyle et d'acétate d'éthyle (1-1 en volumes), 1,2 g de (méthoxy-4 phényl)-2 phényl-4 phénylcarbamoyloxy-5 dihydro-5,6 4H-oxazine-1,3-(4RS, 5RS) fondant à 105° C.

Le (méthoxy-4 phényl)-2 phényl-4 dihydro-5,6 4H-oxazine-1,3 ol-5-(4RS, 5RS) peut être préparé de la manière suivante : à une solution de 5,5 g de N-[dihydroxy-2,3 phényl-1 propyl-(1RS, 2RS)] méthoxy-4 benzamide et de 7,7 cm3 de triéthylamine dans 80 cm3 de dichloro-1,2 éthane maintenue sous atmosphère d'argon, on ajoute à une température voisine de 60° C, 3,82 g de chlorure de tosyle. La solution obtenue est chauffée pendant 16 heures à une température voisine de 60° C puis ajoutée à 50 cm3 d'eau distillée. La phase aqueuse est neutralisée par une solution aqueuse d'acide chlorhydrique 5N puis extraite par 3 fois 50 cm3 de dichlorométhane. Les phases organiques sont réunies, lavées à l'eau, séchées sur sulfate de magnésium, filtrées et concentrées à sec sous pression réduite (2,7 kPa) à 40° C. Le résidu est purifié par chromatographie sur 200 g de silice (0,063 - 0,2 mm) contenus dans une colonne de 3 cm de diamètre, en recueillant des fractions de 25 cm3. Les fractions 3 à 9, éluées par un mélange d'acétate d'éthyle et de cyclohexane (1-1 en volumes), sont réunies et concentrées à sec sous pression réduite (2,7 kPa) à 40° C. On obtient après recristallisation dans l'acétate d'éthyle, 1,41 g de (méthoxy-4 phényl)-2 phényl-4 dihydro-5,6 4H-oxazine-1,3 ol-5-(4RS, 5RS) fondant à 144° C.

Le N-[dihydroxy-2,3 phényl-1 propyl-(1RS, 2RS)] méthoxy-4 benzamide peut être préparé de la manière suivante : en opérant comme à l'exemple 8 pour la préparation du N-[dihydroxy-2,3 phényl-1 propyl-(1RS, 2RS)] indolecarboxamide-2, mais à partir de 3,7 g d'acide méthoxy-4 benzoïque, de 2,26 cm3 de dichlorure d'oxalyle, de 3,7 g d'amino-3 phényl-3 propanediol-1,2-(2RS, 3RS) et de 9,26 cm3 de triéthylamine, on obtient après lavage du précipité à l'eau distillée puis à l'oxyde de diisopropyle, 5,5 g de N-[dihydroxy-2,3 phényl-1 propyl-(1RS, 2RS)] méthoxy-4 benzamide fondant à 178° C.

EXEMPLE 12

A une solution de 0,4 g de diphényl-2,6 dihydro-5,6 4H-oxazine-1,3 ol-5-(5RS, 6SR) dans 10 cm3 de dichloro-1,2 éthane maintenue sous atmosphère d'argon, on ajoute à une température voisine de 20° C, 0,33 g d'isocyanate de phényle. La solution obtenue est agitée à reflux pendant 4 heures puis concentrée à sec sous pression réduite (2,7 kPa). Le résidu est purifié par chromatographie sur 50 g de silice (0,063 - 0,2 mm) contenus dans une colonne de 2 cm de diamètre en recueillant des fractions de 20 cm3. Les fractions 5 à 11, éluées par un mélange d'acétate d'éthyle et de cyclohexane (3-7 en volomes), sont réunies et concentrées à sec sous pression réduite (2,7 kPa) à 40° C. On obtient, après recristallisation dans l'éther de pétrole, 0,36 g de diphényl-2,6 phénylcarbamoyloxy-5 dihydro-5,6 4H-oxazine-1,3-(5RS, 6SR) fondant à 111° C.

Le diphényl-2,6 dihydro-5,6 4H-oxazine-1,3 ol-5-(5RS, 6SR) peut être préparé de la manière suivante : à une solution de 35,5 g de N-cinnamyl benzamide-(E) dans 150 cm3 de dichlorométhane maintenue sous atmosphère d'argon, on ajoute goutte à goutte, à une température voisine de 0° C, 272 cm3 d'une solution 0,55 M d'acide chloro-3 perbenzoïque dans le dichlorométhane. La suspension obtenue est agitée à une température voisine de 0° C pendant 2 heures puis filtrée. Au filtrat maintenu sous atmosphère d'argon, on ajoute goutte à goutte, à une température voisine de 0° C, 21 cm3 d'éthérate de trifluorure de bore. Le mélange est ensuite agité pendant 1 heure à une température voisine de 20° C puis versé dans 150 cm3 d'eau distillée. La phase organique est lavée successivement par 100 cm3 d'une solution aqueuse 1 N de thiosulfate de sodium, 50 cm3 d'eau distillée, 150 cm3 d'une solution aqueuse 1 N de soude, filtrée sur

celite, séchée sur sulfate de magnésium, filtrée puis concentrée à sec sous pression réduite (2,7 kPa) à 40 °C. On obtient, après recristallisation dans l'acétate d'éthyle, 18g de diphényl-2,6 dihydro-5,6 4H-oxazine-1,3 ol-5-(5RS, 6SR) fondant à 135 °C.

Le N-cinnamyl benzamide-(E) peut être préparé selon la méthode décrite par A. PADWA, J.T. BLACKLOCK, Per H.J. CARLSEN et M. PULWER, J. Org. Chem., 44(19), 3281, 1979.

## EXEMPLE 13

A 0,31 g d'une suspension huileuse (50% en poids) d'hydrure de sodium dans 20 cm3 de tétrahydrofuranne anhydre maintenue sous atmosphère d'argon, on ajoute à une température voisine de 20 °C, une solution de 1,52 g de diphényl-2,6 dihydro-5,6 4H-oxazine-1,3 ol-5-(5RS, 6SR) dans 10 cm3 de tétrahydrofuranne anhydre. Le mélange est agité pendant 30 minutes à la même température. On ajoute alors goutte à goutte une solution de 1 g d'isocyanate de chloro-4 phényle dans 10 cm3 de tétrahydrofuranne anhydre et l'on poursuit l'agitation pendant 3 heures à une température voisine de 20 °C. On ajoute ensuite 25 cm3 d'eau distillée et on extrait par 3 fois 50 cm3 de dichlorométhane. Les phases organiques sont réunies, lavées par 3 fois 20 cm3 d'eau distillée, séchées sur sulfate de magnésium et concentrées à sec sous pression réduite (2,7 kPa) à 40 °C. Le résidu est purifié par chromatographie sur 50 g de silice (0,063 - 0,2 mm) contenus dans une colonne de 2 cm de diamètre [éluant : acétate d'éthyle-cyclohexane (2-3 en volumes)] en recueillant des fractions de 20 cm3. Les fractions 4 à 6 sont réunies et concentrées à sec sous pression réduite (2,7 kPa) à 40 °C. On obtient, après recristallisation dans l'oxyde de diisopropyle, 1 g de (chloro-4 phénylcarbamoyloxy)-5 diphényl-2,6 dihydro-5,6 4H-oxazine-1,3-(5RS, 6SR) fondant à 124 °C.

## EXEMPLE 14

A 0,31 g d'une suspension huileuse (50% en poids) d'hydrure de sodium dans 20 cm3 de tétrahydrofuranne anhydre maintenue sous atmosphère d'argon, on ajoute à une température voisine de 20 °C, une solution de 1,52 g de diphényl-2,6 dihydro-5,6 4H-oxazine-1,3 ol-5-(5RS, 6SR) dans 10 cm3 de tétrahydrofuranne anhydre. Le mélange est agité pendant 30 minutes à la même température. On ajoute alors goutte à goutte une solution de 1 g d'isocyanate de chloro-3 phényle dans 10 cm3 de tétrahydrofuranne anhydre et l'on poursuit l'agitation pendant 3 heures à une température voisine de 20 °C. On ajoute ensuite 25 cm3 d'eau distillée et on extrait par 3 fois 50 cm3 de dichlorométhane. Les phases organiques sont réunies, lavées par 3 fois 20 cm3 d'eau distillée, séchées sur sulfate de magnésium et concentrées à sec sous pression réduite (2,7 kPa) à 40 °C. On obtient, après recristallisation dans un mélange d'acétate d'éthyle et d'oxyde de diisopropyle (1-1 en volumes), 0,56 g de (chloro-3 phénylcarbamoyloxy)-5 diphényl-2,6 dihydro-5,6 4H-oxazine-1,3-(5RS, 6SR) fondant à 160 °C.

## EXEMPLE 15

A 0,31 g d'une suspension huileuse (50% en poids) d'hydrure de sodium dans 20 cm3 de tétrahydrofuranne anhydre maintenue sous atmosphère d'argon, on ajoute à une température voisine de 20 °C, une solution de 1,52 g de diphényl-2,6 dihydro-5,6 4H-oxazine-1,3 ol-5-(5RS, 6SR) dans 10 cm3 de tétrahydrofuranne anhydre et on agite la solution obtenue pendant 30 minutes à la même température. On ajoute alors goutte à goutte une solution de 1,24 g d'isocyanate de dichloro-3,4 phényle dans 10 cm3 de tétrahydrofuranne anhydre et l'on poursuit l'agitation pendant 3 heures à une température voisine de 20 °C. On ajoute ensuite 25 cm3 d'eau distillée et on extrait par 3 fois 50 cm3 de dichlorométhane. Les phases organiques sont réunies, lavées par 3 fois 20 cm3 d'eau distillée, séchées sur sulfate de magnésium et concentrées à sec sous pression réduite (2,7 kPa) à 40 °C. Le résidu est purifié par chromatographie sur 50 g de silice (0,063 - 0,2 mm) contenus dans une colonne de 2 cm de diamètre [éluant : acétate d'éthyle-cyclohexane (3-7 en volumes)] en recueillant des fractions de 20 cm3. Les fractions 6 à 9 sont réunies et concentrées à sec sous pression réduite (2,7 kPa) à 40 °C. On obtient ainsi 1,1 g de (dichloro-3,4 phénylcarbamoyloxy)-5 diphényl-2,6 dihydro-5,6 4H-oxazine-1,3-(5RS, 6SR) fondant à 80 °C.

## EXEMPLE 16

A 0,31 g d'une suspension huileuse (50 % en poids) d'hydrure de sodium dans 20 cm3 de tétrahydrofuranne anhydre maintenue sous atmosphère d'argon, on ajoute à une temperature voisine de 20 °C, une solution de 1,52 g de diphényl-2,6 dihydro-5,6 4H-oxazine-1,3 ol-5-(5RS, 6SR) dans 10 cm3 de tétrahydrofuranne anhydre. Le mélange est agité pendant 30 minutes à la même température. On ajoute alors goutte à goutte une solution de 0,9 g d'isocyanate de fluoro-4 phényle dans 10 cm3 de tétrahydrofuranne anhydre et l'on poursuit l'agitation pendant 4 heures à une température voisine de 20 °C. On ajoute ensuite 25 cm3 d'eau distillée et on extrait par 3 fois 50 cm3 de dichlorométhane. Les phases organiques sont réunies, lavées par 3 fois 20 cm3 d'eau distillée, séchées sur sulfate de magnésium et concentrées à sec sous pression réduite (2,7 kPa) à 40 °C. Le résidu est purifié par chromatographie sur 50 g de silice (0,063 - 0,2 mm) contenus dans une colonne de 2 cm de diamètre [éluant ; acétate d'éthyle-cyclohexane (3-7 en volumes)] en recueillant des fractions de 20 cm3. Les fractions 3 à 5 sont réunies et concentrées à sec sous pression réduite (2,7 kpa) à 40 °C. On obtient, après recristallisation dans l'oxyde de diisopropyle, 0,8 g de (fluoro-4 phénylcarbamoyloxy)-5 diphényl-2,6 dihydro-5,6 4H-oxazine-1,3-(5RS, 6SR) fondant à 158 °C.

EXEMPLE 17 -

En opérant d'une manière analogue à celle décrite à l'exemple 16, mais à partir de 0,31 g d'une suspension huileuse (50% en poids) d'hydrure de sodium, de 1,52 g de diphényl-2,6 dihydro-5,6 4H-oxazine-1,3 ol-5-(5RS, 6SR) et de 0,9 g d'isocyanate de fluoro-2 phényle, on obtient, après recristallisation dans l'oxyde de diisopropyle 0,67 g de (fluoro-2 phénylcarbamoyloxy)-5 diphényl-2,6 dihydro-5,6 4H-oxazine-1,3-(5RS, 6SR) fondant à 98 °C.

EXEMPLE 18

A une solution de 1,77 g de diphényl-2,6 dihydro-5,6 4H-oxazine-1,3 ol-5-(5RS, 6SR) et de 0,15 g de diméthylamino-4 pyridine dans 30 cm3 de dichloro-1,2 éthane maintenue sous atmosphère d'argon, on ajoute à une température voisine de 20 °C, 1 g d'isocyanate de méthyl-3 phényle. La solution obtenue est chauffée à reflux pendant 4 heures puis concentrée à sec sous pression réduite (2,7 kPa). Le résidu est purifié par chromatographie sur 50 g de silice (0,063 - 0,2 mm) contenus dans une colonne de 2 cm de diamètre [ éluant : acétate d'éthyle-cyclohexane (3-7 en volumes)], en recueillant des fractions de 20 cm3. Les fractions 5 à 7 sont réunies et concentrées à sec sous pression réduite (2,7 kPa) à 40 °C. On obtient ainsi 1 g de (méthyl-3 phénylcarbamoyloxy)-5 diphényl-2,6 dihydro-5,6 4H-oxazine-1,3-(5RS, 6SR) fondant à 80 °C.

EXEMPLE 19

A une solution de 1,5 g de (méthoxy-4 phényl)-2 phényl-6 dihydro-5,6 4H-oxazine-1,3 ol-5-(5RS, 6SR) dans 20 cm3 de dichloro-1,2 éthane maintenue sous atmosphère d'argon, on ajoute à une température voisine de 20 °C, 1,25 g d'isocyanate de phényle. La solution obtenue est chauffée à reflux pendant 6 heures puis concentrée à sec sous pression réduite (2,7 kPa). Le résidu est purifié par chromatographie sur 50 g de silice (0,063 - 0,2 mm) contenus dans une colonne de 2 cm de diamètre [éluant : cyclohexane-acétate d'éthyle-triéthylamine (60-35-5 en volumes)], en recueillant des fractions de 20 cm3. Les fractions 4 à 10 sont réunies et concentrées à sec sous pression réduite (2,7 kPa) à 40 °C. On obtient, après recristallisation dans l'oxyde de diisopropyle, 0,52 g de (méthoxy-4 phényl)-2 phényl-6 phénylcarbamoyloxy-5 dihydro-5,6 4H-oxazine-1,3-(5RS, 6SR) fondant à 223 °C.

Le (méthoxy-4 phényl)-2 phényl-6 dihydro-5,6 4H-oxazine-1,3 ol-5-(5RS, 6SR) peut être préparé de la manière suivante : à une solution de 4,77 g de N-cinnamyl méthoxy-4 benzamide-(E) dans 30 cm3 de dichlorométhane maintenue sous atmosphère d'argon, on ajoute goutte à goutte, à une température voisine de 0 °C, 37 cm3 d'une solution 0,54 M d'acide chloro-3 perbenzoïque dans le dichlorométhane. La suspension obtenue est agitée à une température voisine de 0 °C pendant 1 heure et 30 minutes puis filtrée. Au filtrat, maintenu sous atmosphère d'argon, on ajoute goutte à goutte, à une temperature voisine de 0 °C, 2,4 cm3 d'éthérate de trifluorure de bore. Le mélange est ensuite agité pendant 1 heure à une température voisine de 20 °C puis hydrolysé avec 19,6 cm3 d'une solution aqueuse 1 N de soude. La phase organique est séparée puis lavée successivement par 30 cm3 d'une solution aqueuse 1 N de

thiosulfate de sodium, 30 cm3 d'eau distillée et 30 cm3 d'une solution aqueuse saturée de bicarbonate de sodium, séchée sur sulfate de magnésium, filtrée puis concentrée à sec sous pression réduite (2,7 kPa) à 40 °C. On obtient, après recristallisation dans un mélange d'acétate d'éthyle et d'oxyde de diisopropyle (1-1 en volumes), 2,6 g de (méthoxy-4 phényl)-2 phényl-6 dihydro-5,6 4H-oxazine-1,3 ol-5-(5RS, 6SR) fondant à 132 °C.

Le N-cinnamyl méthoxy-4 benzamide-(E) peut être préparé de la manière suivante : à une solution de 4,2 g d'acide méthoxy-4 benzoïque et de 0,1 cm3 de diméthylformamide dans 40 cm3 d'éther diéthylique maintenue sous atmosphère d'argon, on ajoute goutte à goutte à une température voisine de 20 °C, 2,6 cm3 de dichlorure d'oxalyle dissous dans 10 cm3 d'éther diéthylique. La solution obtenue est agitée pendant 1 heure et 30 minutes à une température voisine de 20 °C, puis ajoutée goutte à goutte à une solution de 3,3 g de cinnamylamine-(E) et de 7 cm3 de triéthylamine dans 50 cm3 de dichlorométhane, maintenue sous atmosphère d'argon. La suspension est agitée pendant 30 minutes à une température voisine de 30 °C puis versée sur 50 cm3 d'eau distillée. La phase aqueuse est acidifiée jusqu'à un pH voisin de 5 avec une solution aqueuse d'acide chlorhydrique 5N et extraite avec 2 fois 50 cm3 de dichlorométhane. Les phases organiques sont réunies, lavées avec 2 fois 50 cm3 d'eau distillée, séchées sur sulfate de magnésium, filtrées puis concentrées à sec sous pression réduite (2,7 kPa) à 40 °C. On obtient, après recristallisation dans un mélange d'acétate d'éthyle et d'oxyde de diisopropyle (1-1 en volumes), 4,72 g de N-cinnamyl méthoxy-4 benzamide-(E) fondant à 132 °C.

## EXEMPLE 20

A 0,33 g d'une suspension huileuse (50% en poids) d'hy drure de sodium dans 20 cm3 de tétrahydrofuranne anhydre maintenue sous atmosphère d'argon, on ajoute à une température voisine de 20 °C, une solution de 1,8 g de (chloro-4 phényl)-2 phényl-6 dihydro-5,6 4H-oxazine-1,3 ol-5-(5RS, 6SR) dans 10 cm3 de tétrahydrofuranne anhydre. Le mélange est agité 1 heure et 30 minutes à la même température. On ajoute alors goutte à goutte une solution de 0,9 g d'isocyanate de phényle dans 10 cm3 de tétrahydrofuranne anhydre et l'agitation est poursuivie pendant 30 minutes à une température voisine de 20 °C. On ajoute ensuite 25 cm3 d'eau distillée et on extrait par 3 fois 50 cm3 de dichlorométhane. Les phases organiques sont réunies, lavées par 3 fois 20 cm3 d'eau distillée, séchées sur sulfate de magnésium et concentrées à sec sous pression réduite (2,7 kPa) à 40 °C. Le résidu est purifié par chromatographie sur 50 g de silice (0,063 - 0,2 mm) contenus dans une colonne de 2 cm de diamètre [éluant : acétate d'éthyle-cyclohexane (3-7 en volumes)] en recueillant des fractions de 20 cm3. Les fractions 4 à 6 sont réunies et concentrées à sec sous pression réduite (2,7 kPa) à 40 °C. On obtient, après recristallisation dans un mélange d'acétate d'éthyle et d'oxyde de diisopropyle (1-1 en volumes), 1,36 g de (chloro-4 phényl)-2 phényl-6 phénylcarbamoyloxy-5 dihydro-5,6 4H-oxazine-1,3-(5RS, 6SR) fondant à 167 °C.

Le (chloro-4 phényl)-2 phényl-6 dihydro-5,6 4H-oxazine-1,3 ol-5-(5RS, 6SR) peut être préparé de la manière suivante : en opérant comme à l'exemple 19 pour la préparation du (méthoxy-4 phényl)-2 phényl-6 dihydro-5,6 4H-oxazine-1,3 ol-5-(5RS, 6SR), mais à partir de 4,7 g de N-cinnamyl chloro-4 benzamide-(E), on obtient, après recristallisation dans un mélange d'acétate d'éthyle et d'oxyde de diisopropyle (1-1 en volumes), 1,8 g de (chloro-4 phényl)-2 phényl-6 dihydro-5,6 4H-oxazine-1,3 ol-5-(5RS, 6SR) fondant à 155 °C.

Le N-cinnamyl chloro-4 benzamide-(E) peut être préparé de la manière suivante : à une solution de 4,3 g d'acide chloro-4 benzoïque et de 0,1 cm3 de diméthylformamide dans 40 cm3 d'éther diéthylique maintenue sous atmosphère d'argon, on ajoute goutte à goutte à une température voisine de 20 °C, 2,6 cm3 de dichlorure d'oxalyle dissous dans 10 cm3 d'éther diéthylique. La solution obtenue est agitée 1 heure et 30 minutes à une température voisine de 20 °C, puis ajoutée goutte à goutte à une solution de 3,3 g de cinnamylamine-(E) et de 7 cm3 de triéthylamine dans 50 cm3 de dichlorométhane, maintenue sous atmosphère d'argon. La suspension est agitée pendant 30 minutes à une température voisine de 30 °C puis versée dans 20 cm3 d'eau distillée. On obtient, après lavage du précipité ainsi obtenu à l'eau distillée, 4,7 g de N-cinnamyl chloro-4 benzamide-(E) fondant à 143 °C.

## EXEMPLE 21

A une solution de 3,55 g de (chloro-3 phényl)-2 phényl-6 dihydro-5,6 4H-oxazine-1,3 ol-5-(5RS, 6SR) dans 45 cm3 de dichloro-1,2 éthane maintenue sous atmosphère d'argon, on ajoute à une température

voisine de 20 °C, 1,43 g d'isocyanate de phényle. La solution est chauffée à reflux pendant 4 heures puis concentrée à sec sous pression réduite (2,7 kPa). Le résidu est purifié par chromatographie sur 100 g de silice (0,063 - 0,2 mm) contenus dans une colonne de 2,5 cm de diamètre [éluant : cyclohexane-acétate d'éthyle (7-3 en volumes)], en recueillant des fractions de 20 cm3. Les fractions 6 à 11 sont réunies et concentrées à sec sous pression réduite (2,7 kPa) à 40 °C. On obtient, après recristallisation dans l'oxyde de diisopropyle, 2,4 g de (chloro-3 phényl)-2 phényl-6 phénylcarbamoyloxy-5 dihydro-5,6 4H-oxazine-1,3-(5RS, 6SR) fondant à 126 °C.

Le (chloro-3 phényl)-2 phényl-6 dihydro-5,6 4H-oxazine-1,3 ol-5-(5RS, 6SR) peut être préparé de la manière suivante : à une solution de 6,44 g de chloro-3 N-cinnamyl benzamide-(E) dans 50 cm3 de dichlorométhane maintenue sous atmosphère d'argon, on ajoute goutte à goutte, à une température voisine de 0 °C, 49 cm3 d'une solution 0,47 M d'acide chloro-3 perbenzoïque dans le dichlorométhane. La suspension obtenue est agitée à une température voisine de 0 °C pendant 2 heures et 30 minutes puis filtrée. Au filtrat, maintenu sous atmosphère d'argon, on ajoute goutte à goutte, à une température voisine de 0 °C, 3,1 cm3 d'éthérate de trifluorure de bore. Le mélange est ensuite agité pendant 1 heure et 30 minutes à une température voisine de 20 °C puis ajouté à 25 cm3 d'eau distillée. La phase organique est séparée puis lavée successivement par 30 cm3 d'une solution aqueuse 1 N de thiosulfate de sodium, 30 cm3 d'eau distillée et 30 cm3 d'une solution aqueuse saturée de bicarbonate de sodium, séchée sur sulfate de magnésium, filtrée puis concentrée à sec sous pression réduite (2,7 kPa) à 40 °C. On obtient, après recristallisation dans l'oxyde de diisopropyle, 3,55 g de (chloro-3 phényl)-2 phényl-6 dihydro-5,6 4H-oxazine-1,3 ol-5-(5RS, 6SR) fondant à 142 °C.

Le chloro-3 N-cinnamyl benzamide-(E) peut être préparé de la manière suivante : à une solution de 6,8 g d'acide chloro-3 benzoïque et de 0,1 cm3 de diméthylformamide dans 65 cm3 d'éther diéthylique maintenue sous atmosphère d'argon, on ajoute goutte à goutte à une température voisine de 20 °C, 4,1 cm3 de dichlorure d'oxalyle dissous dans 10 cm3 d'éther diéthylique. La solution obtenue est agitée pendant 3 heures à une température voisine de 20 °C, puis ajoutée goutte à goutte à une solution de 5,3 g de cinnamylamine-(E) et de 11,2 cm3 de triéthylamine dans 80 cm3 de dichlorométhane, maintenue sous atmosphère d'argon. La suspension est agitée pendant 30 minutes à une température voisine de 30 °C puis ajoutée à 50 cm3 d'eau distillée. La phase aqueuse est acidifiée jusqu'à un pH voisin de 5 avec une solution aqueuse d'acide chlorhydrique 5N et extraite avec 2 fois 50 cm3 de dichlorométhane. Les phases organiques sont réunies, lavées avec 2 fois 50 cm3 d'eau distillée, séchées sur sulfate de magnésium, filtrées puis concentrées à sec sous pression réduite (2,7 kPa) à 40 °C. On obtient, après recristallisation dans un mélange d'acétate d'éthyle et d'oxyde de diisopropyle (1-16 en volumes), 4,72 g de chloro-3 N-cinnamyl benzamide-(E) fondant à 100 °C.

## EXEMPLE 22

A une solution de 2,3 g de (fluoro-2 phényl)-2 phényl-6 dihydro-5,6 4H-oxazine-1,3 ol-5-(5RS, 6SR) dans 30 cm3 de dichloro-1,2 éthane maintenue sous atmosphère d'argon, on ajoute à one température voisine de 20 °C, 1 g d'isocyanate de phényle. La solution obtenue est agitée à reflux pendant 2 heures puis concentrée à sec sous pression réduite (2,7 kPa). Le résidu est purifié par chromatographie sur 100 g de silice (0,063 - 0,2 mm) contenus dans une colonne de 2,5 cm de diamètre [éluant cyclohexane-acétate d'éthyle (7-3 en volumes)], en recueillant des fractions de 20 cm3. Les fractions 4 à 7 sont réunies et concentrées à sec sous pression réduite (2,7 kPa) à 40 °C. On obtient, après recristallisation dans l'acétate d'éthyle, 1,3 g de (fluoro-2 phényl)-2 phényl-6 phénylcarbamoyloxy-5 dihydro-5,6 4H-oxazine-1,3-(5RS, 6SR) fondant à 128 °C.

Le (fluoro-2 phényl)-2 phényl-6 dihydro-5,6 4H-oxazine-1,3 ol-5-(5RS, 6SR) peut être préparé de la manière suivante : à une solution de 4,8 g de N-cinnamyl fluoro-2 benzamide-(E) dans 45 cm3 de dichlorométhane maintenue sous atmosphère d'argon, on ajoute goutte à goutte, à une température voisine de 0 °C, 47 cm3 d'une solution 0,45 M d'acide chloro-3 perbenzoïque dans le dichlorométhane. La suspension obtenue est agitée à une température voisine de 0 °C pendant 4 heures puis filtrée. Au filtrat, maintenu sous atmosphère d'argon, on ajoute goutte à goutte, à une température voisine de 0 °C, 2,7 cm3 d'éthérate de trifluorure de bore. Le mélange est ensuite agité pendant 2 heures à une température voisine de 20 °C puis ajouté à 25 cm3 d'eau distillée. La phase organique est séparée puis lavée successivement par 30 cm3 d'une solution aqueuse 1 N de thiosulfate de sodium, 30 cm3 d'eau distillée et 30 cm3 d'une solution aqueuse saturée de bicarbonate de sodium, séchée sur sulfate de magnésium, filtrée puis concentrée à sec sous pression réduite (2,7 kPa) à 40 °C. On obtient, après recristallisation dans l'acétate d'éthyle, 2,3 g de (fluoro-2 phényl)-2 phényl-6 dihydro-5,6 4H-oxazine-1,3 ol-5-(5RS, 6SR) fondant à 142 °C.

Le N-cinnamyl fluoro-2 benzamide-(E) peut être préparé en opérant d'une manière analogue à celle décrite à l'exemple 21 pour la préparation du chloro-3 N-cinnamyl benzamide-(E), mais à partir de 5,3 g de cinnamylamine-(E) et de 6,16 g d'acide fluoro-2 benzoïque. On obtient ainsi, après recristallisation dans l'oxyde de diisopropyle, 4,8 g de N-cinnamyl fluoro-2 benzamide-(E) fondant à 68 °C.

EXEMPLE 23

A une solution de 4,7 g de (dichloro-3,4 phényl)-2 phé nyl-6 dihydro-5,6 4H-oxazine-1,3 ol-5-(5RS, 6SR) dans 50 cm3 de dichloro-1,2 éthane maintenue sous atmosphère d'argon, on ajoute à une température voisine de 20 °C, 1,7 g d'isocyanate de phényle. La solution obtenue est chauffée à reflux pendant 3 heures puis concentrée à sec sous pression réduite (2,7 kPa). Le résidu est purifié par chromatographie sur 100 g de silice (0,063 - 0,2 mm) contenus dans une colonne de 2,5 cm de diamètre [éluant : cyclohexane-acétate d'éthyle (7-3 en volumes)], en recueillant des fractions de 20 cm3. Les fractions 2 à 10 sont réunies et concentrées à sec sous pression réduite (2,7 kPa) à 40 °C. On obtient, après recristallisation dans l'oxyde de diisopropyle, 4 g de (dichloro-3,4 phényl)-2 phényl-6 phénylcarbamoyloxy-5 dihydro-5,6 4H-oxazine-1,3-(5RS, 6SR) fondant à 130 °C.

Le (dichloro-3,4 phényl)-2 phényl-6 dihydro-5,6 4H-oxazine-1,3 ol-5-(5RS, 6SR) peut être préparé de la manière suivante : à une solution de 9,6 g de dichloro-3,4 N-cinnamyl benzamide-(E) dans 65 cm3 de dichlorométhane maintenue sous atmosphère d'argon, on ajoute goutte à goutte, à une température voisine de 0 °C, 62 cm3 d'une solution 0,5 M d'acide chloro-3 perbenzoïque dans le dichlorométhane. La suspension obtenue est agitée à une température voisine de 0 °C pendant 3 heures et 30 minutes puis filtrée. Au filtrat maintenu sous atmosphère d'argon, on ajoute goutte à goutte, à une température voisine de 0 °C, 3,9 cm3 d'éthérate de trifluorure de bore. Le mélange est ensuite agité pendant 2 heures à une température voisine de 20 °C puis ajouté à 25 cm3 d'eau distillée. La phase organique est séparée puis lavée successivement par 30 cm3 d'une solution aqueuse 1 N de thiosulfate de sodium, 30 cm3 d'eau distillée et 30 cm3 d'une solution aqueuse saturée de bicarbonate de sodium, séchée sur sulfate de magnésium, filtrée puis concentrée à sec sous pression réduite (2,7 kPa) à 40 °C. On obtient, après recristallisation dans l'acétonitrile, 4,7 g de (dichloro-3,4 phényl)-2 phényl-6 dihydro-5,6 4H-oxazine-1,3 ol-5-(5RS, 6SR) fondant à 158 °C.

Le dichloro-3,4 N-cinnamyl benzamide-(E) peut être préparé en opérant d'une manière analogue à celle décrite à l'exemple 21 pour la préparation du chloro-3 N-cinnamyl benzamide-(E), mais à partir de 5,3 g de cinnamylamine-(E) et de 8,4 g d'acide dichloro-3,4 benzoïque. On obtient ainsi, après recristallisation dans l'oxyde de diisopropyle, 9,6 g de dichloro-3,4 N-cinnamyl benzamide-(E) fondant à 105 °C.

EXEMPLE 24

A une solution de 2,1 g de (fluoro-4 phényl)-2 phényl-6 dihydro-5,6 4H-oxazine-1,3 ol-5-(5RS, 6SR) dans 30 cm3 de dichloro-1,2 éthane maintenue sous atmosphère d'argon, on ajoute à une température voisine de 20 °C, 0,92 g d'isocyanate de phényle. La solution obtenue est chauffée à reflux pendant 4 heures puis concentrée à sec sous pression réduite (2,7 kPa). Le résidu est purifié par chromatographie sur 100 g de silice (0,063 - 0,2 mm) contenus dans une colonne de 2,5 cm de diamètre [éluant : dichlorométhane-méthanol (98-2 en volumes)], en recueillant des fractions de 10 cm3. Les fractions 5 à 15 sont réunies et concentrées à sec sous pression réduite (2,7 kPa) à 40 °C. On obtient ainsi 1,7 g de (fluoro-4 phényl)-2 phényl-6 phénylcarbamoyloxy-5 dihydro-5,6 4H-oxazine-1,3-(5RS, 6SR) fondant à 60 °C.

Le (fluoro-4 phényl)-2 phényl-6 dihydro-5,6 4H-oxazine-1,3 ol-5-(5RS, 6SR) peut être préparé de la manière suivante : à une solution de 4,5 g de N-cinnamyl fluoro-4 benzamide-(E) dans 36 cm3 de dichlorométhane maintenue sous atmosphère d'argon, on ajoute goutte à goutte, à une température voisine de 0 °C, 33 cm3 d'une solution 0,52 M d'acide chloro-3 perbenzoïque dans le dichlorométhane. La suspension est agitée à une température voisine de 0 °C pendant 3 heures et 30 minutes puis filtrée. Au filtrat maintenu sous atmosphère d'argon, on ajoute goutte à goutte, à une température voisine de 0 °C, 3,9 cm3 d'éthérate de trifluorure de bore. Le mélange est ensuite agité pendant 1 heure et 30 minutes à une température voisine de 20 °C puis ajouté à 25 cm3 d'eau distillée. La phase organique est séparée puis lavée successivement par 30 cm3 d'une solution aqueuse 1 N de thiosulfate de sodium, 30 cm3 d'eau distillée et 30 cm3 d'une solution aqueuse saturée de bicarbonate de sodium, séchée sur sulfate de magnésium, filtrée puis concentrée à sec sous pression réduite (2,7 kPa) à 40 °C.

Le résidu est purifié par chromatographie sur 100 g de silice (0,063 - 0,2 mm) contenus dans une

colonne de 2,5 cm de diamètre [éluant : cyclohexane-acétate d'éthyle (7-3 en volumes)], en recueillant des fractions de 20 cm3. Les fractions 9 à 24 sont réunies et concentrées à sec sous pression réduite (2,7 kPa) à 40° C. On obtient ainsi 2,1 g de (fluoro-4 phényl)-2 phényl-6 dihydro-5,6 4H-oxazine-1,3 ol-5-(5RS, 6SR) fondant à 142° C.

Le N-cinnamyl fluoro-4 benzamide-(E) peut être préparé en opérant d'une manière analogue à celle décrite à l'exemple 21 pour la préparation du chloro-3 N-cinnamyl benzamide-(E), mais à partir de 5,3 g de cinnamylamine-(E) et de 8,4 g d'acide fluoro-4 benzoïque, on obtient, après cristallisation dans un mélange de cyclohexane et d'acétate d'éthyle (7-3 en volumes), 4,7 g de N-cinnamyl fluoro-4 benzamide-(E) fondant à 122° C.

EXEMPLE 25

A une solution de 0,86 g de diphényl-2,4 dihydro-5,6 4H-thiazine-1,3 ol-5-(4RS, 5RS) dans 10 cm3 de dichloro-1,2 éthane maintenue sous atmosphère d'argon, on ajoute à une température voisine de 20° C, 1,1 g d'isocyanate de phényle. La solution obtenue est agitée à reflux pendant 3 heures puis concentrée à sec sous pression réduite (2,7 kPa). Le résidu est alors agité avec un mélange d'acétate d'éthyle et de cyclohexane (1-4 en volumes). Un produit insoluble est séparé par filtration et le filtrat concentré à sec sous pression réduite (2,7 kPa). On obtient, après recristallisation dans l'oxyde de diisopropyle, 0,75 g de diphényl-2,4 phénylcarbamoyloxy-5 dihydro-5,6 4H-thiazine-1,3-(4RS, 5RS) fondant à 159° C.

Le diphényl-2,4 dihydro-5,6 4H-thiazine-1,3 ol-5-(4RS, 5RS) peut être préparé de la manière suivante : à une solution de 1,7 g de N-[dihydroxy-2,3 phényl-1 propyl-(1RS, 2RS)] thiobenzamide et de 1.57 g de triphénylphosphine dans 17 cm3 de tétrahydrofuranne anhydre maintenue sous atmosphère d'argon, on ajoute goutte à goutte, à une température voisine de 0° C, une solution de 1,1 g d'azodicarboxylate d'éthyle dissous dans 5 cm3 de tétrahydrofuranne anhydre. La solution obtenue est agitée à une température voisine de 20° C pendant 16 heures puis concentrée à sec sous pression réduite (2,7 kPa). Le résidu est purifié par chromatographie sur 100 g de silice (0,063 - 0,2 mm) contenus dans une colonne de 2,5 cm de diamètre [éluant : cyclohexane-acétate d'éthyle (3-7 en volumes)], en recueillant des fractions de 20 cm3. Les fractions 5 à 11 sont réunies et concentrées à sec sous pression réduite (2,7 kPa) à 40° C. On obtient, après recristallisation dans l'oxyde de diisopropyle, 0,86 g de diphényl-2,4 dihydro-5,6 4H-thiazine-1,3 ol-5-(4RS, 5RS) fondant à 142° C.

Le N-[dihydroxy-2,3 phényl-1 propyl-(1RS, 2RS)] thiobenzamide peut être préparé de la manière suivante : à une solution de 1,05 g d'amino-3 phényl-3 propanediol-1,2-(2RS, 3RS) et de 1,76 cm3 de triéthylamine dans 15 cm3 de dichlorométhane maintenue sous atmosphère d'argon, on ajoute goutte à goutte, à une température voisine de 20° C, une solution de 1 g de chlorure de thiobenzoyle dans 10 cm3 de dichlorométhane. La solution est agitée à une température voisine de 20° C pendant 30 minutes puis hydrolysée avec 20 cm3 d'eau distillée. La phase organique est lavée par 3 fois 20 cm3 d'eau distillée, séchée sur sulfate de magnésium, filtrée puis concentrée à sec sous pression réduite (2,7 kPa) à 40° C. Le résidu est purifié par chromatographie sur 100 g de silice (0,063 -0,2 mm) contenus dans une colonne de 2,5 cm de diamètre [éluant : cyclohexane-acétate d'éthyle (1-1 en volumes)], en recueillant des fractions de 30 cm3. Les fractions 6 à 11 sont réunies et concentrées à sec sous pression réduite (2,7 kPa) à 40° C. On obtient, après cristallisation dans l'acétate d'éthyle, 6 g de N-[dihydroxy-2,3 phényl-1 propyl-(1RS, 2RS)] thiobenzamide fondant à 160° C.

Le chlorure de thiobenzoyle peut être préparé à partir de l'acide dithiobenzoïque selon la méthode décrite par H. VIOLA et R. MAYER, Z. Chem., 15, 348, 1975.

L'acide dithiobenzoïque peut être préparé selon la méthode décrite par D.F. AYCOCK et G.R. JURCH jr, J. Org. Chem., 44(4), 569, 1979.

EXEMPLE 26

En opérant d'une manière analogue à celle décrite à l'exemple 12, mais à partir de 1,55 g de phényl-6 (thiényl-2)-2 dihydro-5,6 4H-oxazine-1,3 ol-5-(5RS, 6SR) et de 0,8 g d'isocyanate de phényle, on obtient après recristallisation dans l'isopropanol, 1,75 g de phényl-6 phénylcarbamoyloxy-5 (thiényl-2)-2 dihydro-5,6 4H-oxazine-1,3-(5RS, 6SR) fondant à 98° C.

Le phényl-6 (thiényl-2)-2 dihydro-5,6 4H-oxazine-1,3 ol-5-(5RS, 6SR) peut être obtenu de la manière suivante : en opérant comme à l'exemple 22 pour la préparation du (fluoro-2 phényl)-2 phényl-6 dihydro-5,6 4H-oxazine-1,3 ol-5-(5RS, 6SR), mais à partir de 5 g de N-cinnamyl thiophènecarboxamide-2-(E), de 38

cm3 d'une solution 0,55 M d'acide chloro-3 perbenzoïque dans le dichlorométhane et de 3,2 g d'éthérate de trifluorure de bore, on obtient, après recristallisation dans le dichlorométhane, 2,9 g de phényl-6 (thiényl-2)-2 dihydro-5,6 4H-oxazine-1,3 ol-5-(5RS, 6SR) fondant à 158 °C.

Le N-cinnamyl thiophènecarboxamide-2-(E) peut être obtenu de la manière suivante : à une solution de 5,3 g de cinnamylamine-(E) dans 80 cm3 de dichloro-1,2 éthane, maintenue sous atmosphère d'azote et à une température voisine de 25 °C, on ajoute 8 g de triéthylamine puis 6,5 g de chlorure de thénoyle. La suspension obtenue est agitée pendant une heure à une température voisine de 25 °C puis versée dans 80 cm3 d'eau. La phase aqueuse est acidifiée jusqu'à un pH voisin de 5 avec une solution aqueuse d'acide chlorhydrique 4N puis extraite par 2 fois 50 cm3 de dichloro-1,2 éthane. Les phases organiques réunies sont lavées par 2 fois 40 cm3 d'eau, séchées sur sulfate de magnésium, filtrées puis concentrées à sec sous pression réduite (4 kPa) à 40 °C. Le résidu est agité dans 40 cm3 d'oxyde d'isopropyle. Le solide est séparé par filtration, lavé par 4 fois 10 cm3 d'oxyde d'isopropyle et séché à l'air. On obtient ainsi 5 g de N-cinnamyl thiophènecarboxamide-2-(E) fondant à 110 °C.

## EXEMPLE 27

En opérant d'une manière analogue à celle décrite à l'exemple 12, mais à partir de 2,6 g de (furyl-3)-2 phényl-6 dihydro-5,6 4H-oxazine-1,3 ol-5-(5RS, 6SR) et de 3,8 g d'isocyanate de phényle et après purification du solide obtenu par chromatographie sur une colonne (hauteur ; 30 cm ; diamètre : 4 cm) de silice (0,2 -0,063 mm) en éluant avec un mélange de dichlorométhane et d'acétate d'éthyle (90-10 en volumes) et en recueillant des fractions de 40 cm3, les fractions 20 à 28 étant réunies et concentrées à sec sous pression réduite (4 kPa) à 45 °C puis recristallisation dans 10 cm3 d'oxyde d'isopropyle, on obtient 1,55 g de (furyl-3)-2 phényl-6 phénylcarbamoyloxy-5 dihydro-5,6 4H-oxazine-1,3-(5RS, 6SR) fondant à 130 °C.

Le (furyl-3)-2 phényl-6 dihydro-5,6 4H-oxazine-1,3 ol-5-(5RS, 6SR) peut être préparé de la manière suivante : en opérant comme à l'exemple 22 pour la préparation du (fluoro-2 phényl)-2 phényl-6 dihydro-5,6 4H-oxazine-1,3 ol-5-(5RS, 6SR), mais à partir de 3,8 g de N-cinnamyl furannecarboxamide-3-(E), de 37 cm3 d'une solution 0,5 M d'acide chloro-3 perbenzoïque dans le dichlorométhane et de 2,7 g d'éthérate de trifluorure de bore, on obtient, après recristallisation dans l'oxyde d'isopropyle, 2,8 g de (furyl-3)-2 phényl-6 dihydro-5,6 4H-oxazine-1,3 ol-5-(5RS, 6SR) fondant à 160 °C.

Le N-cinnamyl furannecarboxamide-3-(E) peut être obtenu de la manière suivante : en opérant comme à l'exemple 21 pour la préparation du chloro-3 N-cinnamyl benzamide-(E), mais à partir de 4 g d'acide furannecarboxylique-3 et de 4 g de cinnamylamine-(E) et après recristallisation dans l'oxyde d'isopropyle, on obtient 3,9 g de N-cinnamyl furannecarboxamide-3-(E) fondant à 115 °C.

## EXEMPLE 28

En opérant d'une manière analogue à celle décrite à l'exemple 12, mais à partir de 0,8 g de diphényl-2,6 méthyl-6 dihydro-5,6 4H-oxazine-1,3 ol-5-(5RS, 6SR) et de 1,8 g d'isocyanate de phényle et après purification du solide obtenu par chromatographie sur une colonne (hauteur : 30 cm ; diamètre ; 2,5 cm) de silice (0,2 - 0,063 mm) en éluant avec on mélange de cyclohexane et d'acétate d'éthyle (80-20 en volumes) et en recueillant des fractions de 20 cm3, les fractions 1 à 5 étant réunies et concentrées à sec sous pression réduite (4 kPa) à 45 °C, puis recristallisation du résidu dans 12 cm3 d'acétonitrile, on obtient 0,95 g de méthyl-6 diphényl-2,6 phénylcarbamoyloxy-5 dihydro-5,6 4H-oxazine-1,3-(5RS, 6SR) fondant à 180 °C.

Le méthyl-6 diphényl-2,6 dihydro-5,6 4H-oxazine-1,3 ol-5-(5RS, 6SR) peut être obtenu de la manière suivante : en opérant comme à l'exemple 22 pour la préparation du (fluoro-2 phényl)-2 phényl-6 dihydro-5,6 4H-oxazine-1,3 ol-5-(5RS, 6SR), mais à partir de 4,2 g N-(phényl-3 butène-2 yl-1) benzamide-(E), de 48 cm3 d'une solution 0,35 M d'acide chloro-3 perbenzoïque dans le dichlorométhane et de 2,6 g d'éthérate de trifluorure de bore et après purification de l'huile obtenue par chromatographie sur une colonne (hauteur : 35 cm ; diamètre : 3 cm) de silice (0,2 - 0,063 mm) en éluant avec un mélange de cyclohexane et d'acétate d'éthyle (60-40 en volumes) et en recueillant des fractions de 30 cm3, les fractions 9 à 17 étant réunies et concentrées à sec sous pression réduite (4 kPa) à 45 °C, puis recristallisation du résidu obtenu dans l'oxyde d'isopropyle, on obtient 1,65 g de méthyl-6 diphényl-2,6 dihydro-5,6 4H-oxazine-1,3 ol-5-(5RS, 6SR) fondant à 130 °C.

Le N-(phényl-3 butène-2 yl-1) benzamide-(E) peut être préparé de la manière suivante ; en opérant comme à l'exemple 26 pour la préparation du N-cinnamyl thiophènecarboxamide-2-(E), mais à partir de 5 g

de phényl-3 butène-2 amine-(E) et de 3,8 g de chlorure de benzoyle et après recristallisation dans l'acétonitrile, on obtient 2,7 g de N-(phényl-3 butène-2 yl-1) benzamide-(E) fondant à 139 °C.

La phényl-3 butène-2 amine-(E) peut être préparée de la manière suivante : à une suspension de 3,45 g d'hydrure d'aluminium et de lithium dans 35 cm3 d'éther diéthylique anhydre, maintenue sous atmosphère d'azote et à une température voisine de -15 °C, on coule, goutte à goutte, une solution de 13 g de phényl-3 butène-2 nitrile (mélange E-Z : 90-10 en moles) dans 100 cm3 d'éther éthylique anhydre. La suspension obtenue est agitée 30 minutes à cette température. On ajoute alors 3,5 cm3 d'eau, 2,7 cm3 de solution aqueuse 4N de soude puis 12,5 cm3 d'eau distillée et on agite 2 heures à une température voisine de 20 °C. Le produit insoluble est séparé par filtration. A la solution éthérée, on ajoute lentement 30 cm3 d'une solution éthérée 3N d'acide chlorhydrique. Le précipité est séparé par filtration, lavé par 2 fois 40 cm3 d'acétonitrile et 2 fois 40 mc3 d'éther éthylique et séché à l'air. On obtient ainsi 5,6 g de chlorhydrate de phényl-3 butène-2 amine-(E) fondant à 215 °C.A une solution de 5,6 g de chlorhydrate de phényl-3 butène-2 amine-(E) dans 60 cm3 d'eau distillée, on ajoute 5 cm3 d'one solution aqueuse 10N de soude puis on extrait l'huile qui s'insolubilise par 2 fois 60 cm3 d'éther diéthylique. Les phases éthérées sont réunies, séchées sur sulfate de magnésium, filtrées puis concentrées à sec sous pression réduite (4 kPa) à 30 °C. On obtient 3,65 g de phényl-3 butène-2 amine-(E) sous forme d'une huile orangée.

Le phényl-3 butène-2 nitrile (Mélange E-Z : 90-10 en moles) peut être préparé selon la méthode décrite par F. TEXIER -BOULLET et A. FOUCAUD, Synthesis, 884, 1979.

EXEMPLE 29

En opérant d'une manière analogue à celle décrite à l'exemple 12, mais à partir de 3,7 g de méthyl-4 diphényl-2,6 dihydro-5,6 4H-oxazine-1,3 ol-5-(mélange des diastéréoisomères 4SR, 5RS, 6SR et 4RS, 5RS, 6SR ; 50-50 en moles) et de 5 g d'isocyanate de phényle puis en purifiant l'huile obtenue par chromatographie sur une colonne (hauteur : 45 cm ; diamètre : 4 cm) de silice en éluant avec un mélange de cyclohexane et d'acétate d'éthyle (85-15 en volumes) et en recueillant des fractions de 25 cm3, les fractions 22 à 25 étant réunies et concentrées à sec sous pression réduite (4 kPa) à 45 °C, puis recristallisation du solide obtenu dans l'acétonitrile, on obtient 0,8 g de diphényl-2,6 méthyl-4 phénylcarbamoyloxy-5 dihydro-5,6 4H-oxazine-1,3-(4SR, 5RS, 6SR) fondant à 150 °C.

Les fractions 28 à 30 sont réunies et concentrées à sec sous pression réduite (4kPa) à 45 °C Le solide obtenu est recristallisé dans l'acétonitrile pour donner 0,1 g de diphényl-2,6 méthyl-4 phénylcarbamoyloxy-5 dihydro-5,6 4H-oxazine-1,3-(4RS, 5RS, 6SR) fondant à 195 °C.

Le méthyl-4 diphényl-2,6 dihydro-5,6 4H-oxazine-1,3 ol-5-(mélange des diastéréoisomères 4SR, SRS, 6SR et 4RS, 5RS, 6SR : 50-50 en moles) peut être obtenu de la manière suivante ; en opérant comme à l'exemple 22 pour la préparation du (fluoro-2 phényl)-2 phényl-6 dihydro-5,6 4H-oxazine-1,3 ol-5-(5RS, 6SR), mais à partir de 4,3 g N-(phényl-4 butène-3 yl-2-(E)-(RS)) benzamide, de 34,5 cm3 d'une solution 0,5 M d'acide chloro-3 perbenzoïque dans le dichlorométhane et de 2,7 g d'éthérate de trifluorure de bore, on obtient 3,8 g de méthyl-4 diphényl-2,6 dihydro-5,6 4H-oxazine-1,3 ol-5-(mélange des diastéréoisomères 4SR, 5RS, 6SR et 4RS, 5RS, 6SR : 50-50 en moles) fondant à 60 °C.

Le N-(phényl-4 butène-3 yl-2-(E)-(RS)) benzamide peut être préparé de la manière suivante : en opérant comme à l'exemple 26 pour la préparation du N-cinnamyl thiophènecarboxamide-2-(E), mais à partir de 4,6 g de (phényl-4 butène-3 yl-2) amine-(E)-(RS) et de 5,3 g de chlorure de benzoyle et après agitation du résidu dans l'oxyde d'isopropyle, on obtient 4,3 g de N-(phényl-4 butène-3 yl-2-(E)-(RS)) benzamide fondant à 135 °C.

La (phényl-4 butène-3 yl-2) amine-(E)-(RS) peut être préparée selon la méthode décrite par T.G. SCHENCK et B. BOSNICH, J. Am. Chem. Soc., 107(7), 2064, 1985.

EXEMPLE 30

A une solution de 0,84 g de (nitro-4 phénoxycarbonyloxy)-5 diphényl-2,4 dihydro-5,6 4H-oxazine-1,3-(4RS, 5RS) et de 0,15 g de diméthylamino-4 pyridine dans 25 cm3 d'acétonitrile maintenue sous atmosphère d'argon, on ajoute à une température voisine de 20 °C, 0,55 g de méthylènedioxy-3,4 aniline. La solution obtenue est chauffée à reflux pendant 3 heures puis concentrée à sec sous pression réduite (2,7 kPa). Le résidu est purifié par chromatographie sur 30 g de silice (0,063 - 0,2 mm) contenus dans une colonne de 1 cm de diamètre en recueillant des fractions de 20 cm3. Les fractions 1 à 8, éluées par un mélange d'acétate d'éthyle et de cyclohexane (3-7 en volumes), sont réunies et concentrées à sec sous

pression réduite (2,7 kPa) à 40 °C. On obtient, après recristallisation dans un mélange d'acétonitrile et d'oxyde de diisopropyle (1-9 en volumes), 0,49 g de (méthylènedioxy-3,4 phénylcarbamoyloxy)-5 diphényl-2,4 dihydro-5,6 4H-oxazine-1,3-(4RS, 5RS) fondant à 163 °C.

Le (nitro-4 phénoxycarbonyloxy)-5 diphényl-2,4 dihydro-5,6 4H-oxazine-1,3-(4RS, 5RS) peut être préparé de la manière suivante : à une solution de 15,2 g de diphényl-2,4 dihydro-5,6 4H-oxazine-1,3 ol-5-(4RS,5RS) dans 100 cm3 de pyridine maintenue sous atmosphère d'argon, on ajoute à une température voisine de 0 °C, 24 g de chloroformiate de nitro-4 phényle. La solution obtenue est agitée pendant 16 heures à une température voisine de 20 °C puis versée dans 750 cm3 d'eau distillée. Le mélange est extrait par 3 fois 200 cm3 de dichlorométhane. Les phases organiques sont réunies, lavées par 2 fois 100 cm3 d'eau distillée, séchées sur sulfate de magnésium, filtrées puis concentrées à sec sous pression réduite (2,7 kPa) à 40 °C. Le résidu huileux est purifié par chromatographie sur 400 g de silice (0,063 - 0,2 mm) contenus dans une colonne de 7 cm de diamètre en recueillant des fractions de 25 cm3. Les fractions 1 à 15, éluées par un mélange d'acétate d'éthyle et de cyclohexane (3-7 en volumes), sont concentrées à sec sous pression réduite (2,7 kPa) à 40 °C. On obtient, après recristallisation dans un mélange d'acétate d'éthyle et d'oxyde de diisopropyle (4-1 en volumes), 18,5 g de (nitro-4 phénoxycarbonyloxy)-5 diphényl-2,4 dihydro-5,6 4H-oxazine-1,3-(4RS, 5RS) fondant à 126 °C.

EXEMPLE 31

A une solution de 1,67 g de (nitro-4 phénoxycarbonyloxy)-5 diphényl-2,6 dihydro-5,6 4H-oxazine-1,3-(5RS, 6SR) et de 0,2 g de diméthylamino-4 pyridine dans 50 cm3 d'acétonitrile anhydre maintenue sous atmosphère d'argon, on ajoute à une température voisine de 20 °C, 1,1 g de méthylènedioxy-3,4 aniline. La solution obtenue est chauffée à reflux pendant 5 heures puis concentrée à sec sous pression réduite (2,7 kPa). L'huile obtenue est purifiée par chromatographie sur 50 g silice (0,063 - 0,2 mm) contenus dans une colonne de 2 cm de diamètre [éluant : cyclohexane-acétate d'éthyle-triéthylamine (65-30-5 en volumes)], en recueillant des fractions de 15 cm3. Les fractions 3 à 6 sont réunies et concentrées à sec sous pression réduite (2,7 kPa) à 40 °C. On obtient, après recristallisation dans l'oxyde de diisopropyle, 0,8 g de (méthylènedioxy-3,4 phénylcarbamoyloxy)-5 diphényl-2,6 dihydro-5,6 4H-oxazine-1,3-(5RS, 6SR) fondant à 138 °C.

Le (nitro-4 phénoxycarbonyloxy)-5 diphényl-2,6 dihydro-5,6 4H-oxazine-1,3-(5RS, 6SR) peut être préparé de la manière suivante : à une solution de 30,3 g de diphényl-2,6 dihydro-5,6 4H-oxazine-1,3 ol-5-(5RS, 6SR) dans 300 cm3 de pyridine anhydre maintenue sous atmosphère d'argon, on ajoute à une température voisine de 0 °C, 36 g de chloroformiate de nitro-4 phényle. La solution obtenue est agitée pendant 16 heures à une température voisine de 20 °C puis versée dans 2000 cm3 d'eau distillée. Le mélange est extrait par 3 fois 500 cm3 de dichlorométhane. Les phases organiques sont réunies, lavées par 3 fois 400 cm3 d'eau distillée, séchées sur sulfate de magnésium, filtrées puis concentrées à sec sous pression réduite (2,7 kPa) à 40 °C. On obtient, après recristallisation dans un mélange d'acétate d'éthyle et d'oxyde de diisopropyle (3-2 en volumes), 30 g de (nitro-4 phénoxycarbonyloxy)-5 diphényl-2,6 dihydro-5,6 4H-oxazine-1,3-(5RS, 6SR) fondant à 160 °C.

EXEMPLE 32

A une solution de 3 g de (nitro-4 phénoxycarbonyloxy)-5 diphényl-2,6 dihydro-5,6 4H-oxazine-1,3-(5RS, 6SR) et de 0,3 g de diméthylamino-4 pyridine dans 100 cm3 d'acétonitrile anhydre maintenue sous atmosphère d'argon, on ajoute à une température voisine de 20 °C, 1,7 g de diméthyl-3,4 aniline. La solution obtenue est chauffée à reflux pendant 4 heures puis concentrée à sec sous pression réduite (2,7 kPa). Le résidu est purifié par chromatographie sur 50 g silice (0,063 - 0,2 mm) contenus dans une colonne de 2 cm de diamètre [éluant : cyclohexane-acétate d'éthyle-triéthylamine (65-30-5 en volumes)], en recueillant des fractions de 25 cm3. Les fractions 2 à 6 sont réunies et concentrées à sec sous pression réduite (2,7 kPa) à 40 °C. On obtient ainsi 1 g de (diméthyl-3,4 phénylcarbamoyloxy)-5 diphényl-2,6 dihydro-5,6 4H-oxazine-1,3-(5RS, 6SR) fondant à 80 °C.

EXEMPLE 33

A une solution de 3 g de (nitro-4 phénoxycarbonyloxy)-5 diphényl-2,6 dihydro-5,6 4H-oxazine-1,3-(5RS,

6SR) et de 0,3 g de diméthylamino-4 pyridine dans 50 cm3 de N,N-diméthylformamide anhydre maintenue sous atmosphère d'argon, on ajoute à une température voisine de 20 °C, 1,72 g de méta-anisidine. La solution obtenue est chauffée à une température voisine de 70 °C pendant 6 heures puis concentrée à sec sous pression réduite (2,7 kPa). Le résidu est purifié par chromatographie sur 50 g silice (0,063 - 0,2 mm) contenus dans une colonne de 2 cm de diamètre [éluant : cyclohexane-acétate d'éthyle-triéthylamine (65-30-5 en volumes)], en recueillant des fractions de 25 cm3. Les fractions 2 à 5 sont réunies et concentrées à sec sous pression réduite (2,7 kPa) à 40 °C. On obtient, après recristallisation dans l'oxyde de diisopropyle, 1,8 g de (méthoxy-3 phénylcarbamoyloxy)-5 diphényl-2,6 dihydro-5,6 4H-oxazine-1,3-(5RS, 6SR) fondant à 110 °C.

EXEMPLE 34

En opérant d'une manière analogue à celle décrite à l'exemple 33, mais à partir de 3 g de (nitro-4 phénoxycarbonyloxy)-5 diphényl-2,6 dihydro-5,6 4H-oxazine-1,3-(5RS, 6SR) et de 1,9 g de nitro-3 aniline, on obtient, après recristallisation dans un mélange d'acétonitrile et d'oxyde de diisopropyle (1-1 en volumes), 0,85 g de (nitro-3 phénylcarbamoyloxy)-5 diphényl-2,6 dihydro-5,6 4H-oxazine-1,3-(5RS, 6SR) fondant à 176 °C.

EXEMPLE 35

A une solution de 1,34 g de (méthoxy-4 phényl)-2 (nitro-4 phénoxycarbonyloxy)-5 phényl-4 dihydro-5,6 4H-oxazine-1,3-(4RS, SRS) et de 0,1 g de diméthylamino-4 pyridine dans 30 cm3 d'acétonitrile anhydride maintenue sous atmosphère d'argon, on ajoute, à une température voisine de 20 °C, 0,82 g de méthylènedioxy-3,4 aniline. La solution obtenue est chauffée à reflux pendant 4 heures puis concentrée à sec sous pression réduite (2,7 kPa). Le résidu est purifié par chromatographie sur 30 g de silice (0,063 - 0,2 mm) contenus dans une colonne de 2 cm de diamètre [éluant ; cyclohexane-acétate d'éthyle-triéthylamine (65-35-5 en volumes)], en recueillant des fractions de 25 cm3. Les fractions 2 à 5 sont réunies et concentrées à sec sous pression réduite (2,7 kPa) à 40 °C. On obtient, après recristallisation dans un mélange d'acétate d'éthyle et d'oxyde de diisopropyle (1-2 en volumes), 1,4 g de (méthoxy-4 phényl)-2 (méthylènedioxy-3,4 phénylcarbamoyloxy)-5 phényl-4 dihydro-5,6 4H-oxazine-1,3-(4RS, 5RS) fondant à 160 °C.

Le (méthoxy-4 phényl)-2 (nitro-4 phénoxycarbonyloxy)-5 phényl-4 dihydro-5,6 4H-oxazine-1,3-(4RS, 5RS) peut être préparé de la manière suivante : à une solution de 6 g de (méthoxy-4 phényl)-2 phényl-4 dihydro-5,6 4H-oxazine-1,3 ol-5-(4RS,5RS) dans 75 cm3 de pyridine anhydride maintenue sous atmosphère d'argon, on ajoute à une température voisine de 0 °C, 6,4 g de chloroformiate de nitro-4 phényle. La solution obtenue est agitée pendant 5 heures à une température voisine de 20 °C puis versée dans 400 cm3 d'eau distillée. Le mélange est extrait par 3 fois 250 cm3 de dichlorométhane. Les phases organiques sont réunies, lavées par 3 fois 150 cm3 d'eau distillée, séchées sur sulfate de magnésium, filtrées puis concentrées à sec sous pression réduite (2,7 kPa) à 40 °C. On obtient, après recristallisation dans l'acétate d'éthyle, 5 g de (méthoxy-4 phényl)-2 (nitro-4 phénoxycarbonyloxy)-5 phényl-4 dihydro-5,6 4H-oxazine-1,3-(4RS, 5RS) fondant à 164 °C.

EXEMPLE 36

A une solution de 4,1 g de (nitro-4 phényl)-2 phényl-6 dihydro-5,6 4H-oxazine-1,3 ol-5-(5RS, 6SR) dans 45 cm3 de dichloro-1,2 éthane maintenue sous atmosphère d'argon, on ajoute à une température voisine de 20 °C, 1,6 g d'isocyanate de phényle. La solution obtenue est chauffée à reflux pendant 4 heures puis concentrée à sec sous pression réduite (2,7 kPa). Le résidu est purifié par chromatographie sur 100 g de silice (0,063 - 0,2 mm) contenus dans une colonne de 2,5 cm de diamètre [éluant ; dichlorométhane-méthanol (97-3 en volumes)], en recueillant des fractions de 20 cm3. Les fractions 10 à 31 sont réunies et concentrées à sec sous pression réduite (2,7 kPa) à 40 °C. On obtient, après recristallisation dans l'acétonitrile, 1,35 g de (nitro-4 phényl)-2 phényl-6 phénylcarbamoyloxy-5 dihydro-5,6 4H-oxazine-1,3-(5RS, 6SR) fondant à 170 °C.

Le (nitro-4 phényl)-2 phényl-6 dihydro-5,6 4H-oxazine-1,3 ol-5-(5RS, 6SR) peut être préparé de la manière suivante : à une solution de 5,6 g de N-cinnamyl nitro-4 benzamide-(E) dans 45 cm3 de

dichlorométhane maintenue sous atmosphère d'argon, on ajoute goutte à goutte, à une température voisine de 0 °C, 45 cm3 d'une solution 0,48 M d'acide chloro-3 perbenzoïque dans le dichlorométhane. La suspension obtenue est agitée à une temperature voisine de 0 °C pendant 1 heure puis filtrée. Au filtrat maintenu sous atmosphère d'argon, on ajoute goutte à goutte, à une température voisine de 0 °C, 2,5 cm3 d'éthérate de trifluorure de bore. Le mélange est ensuite agité pendant 2 heures et 30 minutes à une température voisine de 20 °C puis versé dans 100 cm3 d'eau distillée. La phase organique est séparée puis lavée successivement par 30 cm3 d'une solution aqueuse 1 N de thiosulfate de sodium, 30 cm3 d'eau distillée, 30 cm3 d'une solution aqueuse saturée de bicarbonate de sodium, séchée sur sulfate de magnésium, filtrée puis concentrée à sec sous pression réduite (2,7 kPa) à 40 °C. On obtient ainsi 4,4 g de (nitro-4 phényl)-2 phényl-6 dihydro-5,6 4H-oxazine-1,3 ol-5-(5RS, 6SR) fondant à 148 °C.

Le N-cinnamyl nitro-4 benzamide-(E) peut être préparé selon la méthode décrite par S. P. Mc MANUS, Don W. WARE, A. RANDY, J. Org. Chem., 43(22), 4288, 1978.

EXEMPLE 37

A une solution de 1,4 g de (méthyl-4 phényl)-2 phényl-6 dihydro-5,6 4H-oxazine-1,3 ol-5-(5RS, 6SR) dans 20 cm3 de dichloro-1,2 éthane maintenue sous atmosphère d'argon, on ajoute à une température voisine de 20 °C, 0,61 g d'isocyanate de phényle. La solution obtenue est chauffée à reflux pendant 5 heures puis concentrée à sec sous pression réduite (2,7 kPa). Le résidu est purifié par chromatographie sur 50 g de silice (0,063 - 0,2 mm) contenus dans une colonne de 2,5 cm de diamètre [éluant : dichlorométhane-méthanol (9-1 en volumes)], en recueillant des fractions de 20 cm3. Les fractions 7 à 17 sont réunies et concentrées à sec sous pression réduite (2,7 kPa) à 40 °C. On obtient ainsi 0,7 g de (méthyl-4 phényl)-2 phényl-6 phénylcarbamoyloxy-5 dihydro-5,6 4H-oxazine-1,3-(5RS, 6SR) fondant à 138 °C.

Le (méthyl-4 phényl)-2 phényl-6 dihydro-5,6 4H-oxazine-1,3 ol-5-(5RS, 6SR) peut être préparé de la manière suivante : à une solution de 4,2 g de N-cinnamyl méthyl-4 benzamide-(E) dans 40 cm3 de dichlorométhane maintenue sous atmosphère d'argon, on ajoute goutte à goutte, à une température voisine de 0 °C, 41 cm3 d'une solution 0,45 M d'acide chloro-3 perbenzoïque dans le dichlorométhane. La suspension obtenue est agitée à une température voisine de 0 °C pendant 2 heures puis filtrée. Au filtrat maintenu sous atmosphère d'argon, on ajoute goutte à goutte, à une température voisine de 0 °C, 2,1 cm3 d'éthérate de trifluorure de bore. Le mélange est ensuite agité pendant 2 heures à une température voisine de 20 °C puis versé dans 100 cm3 d'eau distillée. La phase organique est séparée puis lavée successivement par 30 cm3 d'une solution aqueuse 1 N de thiosulfate de sodium, 30 cm3 d'eau distillée, 30 cm3 d'une solution aqueuse saturée de bicarbonate de sodium, séchée sur sulfate de magnésium, filtrée puis concentrée à sec sous pression réduite (2,7 kPa) à 40 °C. Le résidu est purifié par chromatographie sur 50 g de silice (0,063 - 0,2 mm) contenus dans une colonne de 2,5 cm de diamètre [éluant : dichlorométhane-méthanol (98-2 en volumes)], en recueillant des fractions de 20 cm3. Les fractions 19 à 25 sont réunies et concentrées à sec sous pression réduite (2,7 kPa) à 40 °C. On obtient, après recristallisation dans l'acétate d'éthyle, 1,4 g de (méthyl-4 phényl)-2 phényl-6 dihydro-5,6 4H-oxazine-1,3 ol-5-(5RS, 6SR) fondant à 168 °C.

Le N-cinnamyl méthyl-4 benzamide-(E) peut être préparé en opérant d'une manière analogue à celle décrite à l'exemple 21 pour la préparation du chloro-3 N-cinnamyl benzamide-(E) mais à partir de 5,3 g de cinnamylamine et de 6 g d'acide méthyl-4 benzoïque. On obtient ainsi, après recristallisation dans l'acétate d'éthyle, 4,2 g de N-cinnamyl méthyl-4 benzamide-(E) fondant à 134 °C.

EXEMPLE 38

A une solution de 1 g de (diméthylamino-4 phényl)-2 phényl-6 dihydro-5,6 4H-oxazine-1,3 ol-5-(5RS, 6SR) dans 12 cm3 de dichloro-1,2 éthane maintenue sous atmosphère d'argon, on ajoute à une température voisine de 20 °C, 0,49 g d'isocyanate de phényle. La solution obtenue est chauffée à reflux pendant 2 heures puis concen trée à sec sous pression réduite (2,7 kPa). Le résidu est purifié par chromatographie sur 50 g de silice (0,063 - 0,2 mm) contenus dans une colonne de 2,5 cm de diamètre [éluant : cyclohexane-acétate d'éthyle (7-3 en volumes)], en recueillant des fractions de 20 cm3. Les fractions 9 à 15 sont réunies et concentrées à sec sous pression réduite (2,7 kPa) à 40 °C. On obtient, après recristallisation dans l'oxyde de diéthyle, 0,55 g de (diméthylamino-4 phényl)-2 phényl-6 phénylcarbamoyloxy-5 dihydro-5,6 4H-oxazine-1,3-(5RS, 6SR) fondant à 183 °C.

Le (diméthylamino-4 phényl)-2 phényl-6 dihydro-5,6 4H-oxazine-1,3 ol-5-(5RS, 6SR) peut être préparé de la manière suivante : à une solution de 2,8 g de N-(époxy-2,3 phényl-3 propyl) diméthylamino-4 benzamide-(2RS, 3RS) dans 65 cm3 de dichlorométhane maintenue sous atmosphère d'argon, on ajoute goutte à goutte, à une température voisine de 0° C, 1,3 cm3 d'éthérate de trifluorure de bore dissous dans 3 cm3 de dichlorométhane. La solution obtenue est ensuite agitée pendant 1 heure à une température voisine de 20° C puis additionnée de 11 cm3 d'une solution aqueuse 1 N de soude en maintenant l'agitation pendant 30 minutes. La phase aqueuse est séparée puis réextraite par 2 fois 50 cm3 de dichlorométhane. Les phases organiques sont réunies, séchées sur sulfate de magnésium, filtrées puis concentrées à sec sous pression réduite (2,7 kPa) à 40° C. Le résidu est purifié par chromatographie sur 200 g de silice (0,063 - 0,2 mm) contenus dans une colonne de 3 cm de diamètre (éluant : acétate d'éthyle), en recueillant des fractions de 35 cm3. Les fractions 6 à 31 sont réunies et concentrées à sec sous pression réduite (2,7 kPa) à 40° C. On obtient ainsi 1,23 g de (diméthylamino-4 phényl)-2 phényl-6 dihydro-5,6 4H-oxazine-1,3 ol-5-(5RS, 6SR) fondant à 172° C.

Le N-(époxy-2,3 phényl-3 propyl) diméthylamino-4 benzamide-(2RS, 3RS) peut être préparé de la manière suivante :

a- Préparation de la solution A :

A une solution de 3,3 g d'acide diméthylamino-4 benzoïque dans 60 cm3 de dichlorométhane maintenue sous atmosphère d'argon, on ajoute goutte à goutte, à une température voisine de 0° C, 3,24 g de carbonyldiimidazole. La solution obtenue (solution A) est ensuite agitée pendant 2 heures à une température voisine de 20° C pendant que l'on prépare la solution B.

b- Préparation de la solution B :

4,9 g de N-(époxy-2,3 phényl-3 propyl) trifluoroacétamide-(2RS, 3RS) sont ajoutés à 6 cm3 d'une solution aqueuse 3,57 N de potasse. Après 7 minutes d'agitation à une température voisine de 20° C, la solution obtenue est saturée avec du chlorure de sodium puis extraite par 2 fois 50 cm3 de dichlorométhane. Les phases organiques sont réunies, séchées sur sulfate de magnésium puis filtrées, constituant ainsi une solution chlorométhylénique d'époxy-2,3 phényl-3 propanamine (2RS, 3RS) (solution B).

La solution B est ajoutée goutte à goutte à la solution A. La solution obtenue est ensuite agitée pendant 3 heures à une température voisine de 20° C puis versée dans 100 cm3 d'eau distillée. La phase aqueuse est séparée puis réextraite par 2 fois 50 cm3 de dichlorométhane. Les phases organiques sont réunies, lavées par une solution aqueuse saturée en chlorure de sodium, séchées sur sulfate de magnésium, filtrées puis concentrées à sec sous pression réduite (2,7 kPa) à 40° C. On obtient, après recristallisation dans un mélange d'acétate d'éthyle et d'oxyde de diisopropyle (2-1 en volumes), 2,95 g de N-(époxy-2,3 phényl-3 propyl) diméthylamino-4 benzamide-(2RS, 3RS) fondant à 127° C.

Le N-(époxy-2,3 phényl-3 propyl) trifluoroacétamide-(2RS, 3RS) peut être préparé de la manière suivante : à une solution de 17 g de N-cinnamyl trifluoroacétamide-(E) dans 150 cm3 de dichlorométhane maintenue sous atmosphère d'argon, on ajoute goutte à goutte, à une température voisine de 0° C, 180 cm3 d'une solution 0,425 M d'acide chloro-3 perbenzoïque dans le dichlorométhane. La suspension obtenue est agitée à une température voisine de 20° C pendant 6 heures, refroidie à une température voisine de 0° C, puis filtrée. Le filtrat est lavé successivement par 200 cm3 d'une solution aqueuse 1 N de thiosulfate de sodium, 200 cm3 d'eau distillée, 200 cm3 d'une solution aqueuse saturée de bicarbonate de sodium, séché sur sulfate de magnésium, filtré puis concentré à sec sous pression réduite (2,7 kPa) à 40° C. On obtient ainsi 18 g de N-(époxy-2,3 phényl-3 propyl) trifluoroacétamide-(2RS, 3RS) fondant à 79° C.

Le N-cinnamyl trifluoroacétamide-(E) peut être préparé selon la méthode décrite par P. HODGE et al., Synthesis, 1984, 941.

EXEMPLE 39

A une solution de 1,55 g d'(acétamido-4 phényl)-2 phényl-6 dihydro-5,6 4H-oxazine-1,3 ol-5-(5RS, 6SR) dans 20 cm3 de dichloro-1,2 éthane maintenue sous atmosphère d'argon, on ajoute à une température voisine de 20° C, 0,72 g d'isocyanate de phényle. La solution obtenue est chauffée à 80° C pendant 50

heures puis concentrée à sec sous pression réduite (2,7 kPa). Le solide obtenu est purifié par chromatographie sur 100 g de silice (0,063 - 0,2 mm) contenus dans une colonne de 2,5 cm de diamètre en recueillant des fractions de 30 cm3. Les fractions 8 à 15 sont éluées par un mélange d'acétate d'éthyle et de cyclohexane (3-2 en volumes) puis concentrées à sec sous pression réduite (2,7 kPa) à 40 °C. On obtient, après recristallisation dans l'acétate d'éthyle, 1,25 g d'(acétamido-4 phényl)-2 phényl-6 phénylcarbamoyloxy-5 dihydro-5,6 4H-oxazine-1,3-(5RS, 6SR) fondant entre 214 et 220 °C.

L' (acétamido-4 phényl)-2 phényl-6 dihydro-5,6 4H-oxazine-1,3 ol-5-(5RS, 6SR) peut être préparé de la manière suivante : à une solution de 4,5 g de N-(époxy-2,3 phényl-3 propyl) acétamido-4 benzamide-(2RS, 3RS) dans 80 cm3 de dichlorométhane maintenue sous atmosphère d'argon, on ajoute goutte à goutte, à une température voisine de 0 °C, 2 cm3 d'éthérate de trifluorure de bore dissous dans 5 cm3 de dichlorométhane. La solution obtenue est ensuite agitée pendant 30 minutes à une température voisine de 20 °C puis additionnée de 16 cm3 d'une solution aqueuse 1 N de soude en maintenant l'agitation pendant 30 minutes. La phase aqueuse est séparée puis réextraite par 2 fois 50 cm3 d'acétate d'éthyle. Les phases organiques sont réunies, séchées sur sulfate de magnésium, filtrées puis concentrées à sec sous pression réduite (2,7 kPa) à 40 °C. On obtient, après recristallisation dans un mélange d'éther de pétrole et d'éthanol (95-5 en volumes), 2 g d'(acétamido-4 phényl)-2 phényl-6 dihydro-5,6 4H-oxazine-1,3 ol-5-(5RS, 6SR) fondant à 220 °C.

Le N-(époxy-2,3 phényl-3 propyl) acétamido-4 benza mide-(2RS, 3RS) peut être préparé de la manière suivante :

a- Préparation de la solution A :

A une solution de 7 g d'acide acétamido-4 benzoïque dans 80 cm3 de dichlorométhane maintenue sous atmosphère d'argon, on ajoute par petites portions à une température voisine de 0 °C, 6,5 g de carbonyldiimidazole. La solution obtenue (solution A) est ensuite agitée pendant 2 heures à une température voisine de 20 °C pendant que l'on prépare la solution B.

b- Préparation de la solution B :

11 g de N-(époxy-2,3 phényl-3 propyl) trifluoroacétamide-(2RS, 3RS) sont ajoutés à 12,5 cm3 d'une solution aqueuse 3,6 N de potasse. Après 7 minutes d'agitation à une température voisine de 20 °C, la solution obtenue est saturée avec du chlorure de sodium puis extraite par 2 fois 100 cm3 de dichlorométhane. Les phases organiques sont réunies, séchées sur sulfate de magnésium puis filtrées, constituant ainsi une solution chlorométhylénique d'époxy-2,3 phényl-3 propanamine (2RS, 3RS) (solution B).

La solution B est ajoutée goutte à goutte à la solution A. La solution obtenue est ensuite agitée pendant 3 heures à une température voisine de 20 °C puis versée dans 100 cm3 d'eau distillée. La phase aqueuse est séparée puis réextraite par 2 fois 50 cm3 de dichlorométhane. Les phases organiques sont réunies, lavées par une solution aqueuse saturée en chlorure de sodium, séchées sur sulfate de magnésium, filtrées puis concentrées à sec sous pression réduite (2,7 kPa) à 40 °C. On obtient, après recristallisation dans l'acétate d'éthyle 4,8 g de N-(époxy-2,3 phényl-3 propyl) acétamido-4 benzamide-(2RS,3RS).

EXEMPLE 40

A une solution de 0,8 g d'(éthoxy-4 phényl)-2 phényl-6 dihydro-5,6 4H-oxazine-1,3 ol-5-(5RS, 6SR) dans 10 cm3 de dichloro-1,2 éthane maintenue sous atmosphère d'argon, on ajoute à une température voisine de 20 °C, 0,38 g d'isocyanate de phényle. La solution obtenue est chauffée à 80 °C pendant 3 heures puis concentrée à sec sous pression réduite (2,7 kPa). Le solide obtenu est purifié par chromatographie sur 100 g de silice (0,063 - 0,2 mm) contenus dans une colonne de 2 cm de diamètre en recueillant des fractions de 20 cm3. Les fractions 3 et 4, éluées par un mélange de cyclohexane et d'acétate d'éthyle (6-4 en volumes), sont concentrées à sec sous pression réduite (2,7 kPa) à 40 °C. Le solide obtenu est à nouveau purifié par chromatographie sur 100 g de silice (0,063 - 0,2 mm) contenus dans une colonne de 2 cm de diamètre en recueillant des fractions de 20 cm3. Les fractions 5 à 10, éluées par un mélange de cyclohexane, d'acétate d'éthyle et de triéthylamine (65-30-5 en volumes), sont concentrées à sec sous pression réduite (2,7 kPa) à 40 °C. On obtient, après recristallisation dans l'acétate d'éthyle, 0,35 g d'-(éthoxy-4 phényl)-2 phényl-6 phénylcarbamoyloxy-5 dihydro-5,6 4H-oxazine-1,3-(5RS, 6SR) fondant à

80 °C.

L'(éthoxy-4 phényl)-2 phényl-6 dihydro-5,6 4H-oxazine-1,3 ol-5-(5RS, 6SR) peut être préparé de la manière suivante : à une solution de 5,7 g d'éthoxy-4 N-cinnamyl benzamide-(E) dans 75 cm3 de dichlorométhane maintenue sous atmosphère d'argon, on ajoute goutte à goutte, à une température voisine de 0 °C, 46,5 cm3 d'une solution 0,43 M d'acide chloro-3 perbenzoïque dans le dichlorométhane. La suspension obtenue est agitée à une température voisine de 20 °C pendant 2 heures puis filtrée. Au filtrat obtenu est maintenu sous atmosphère d'argon, on ajoute goutte à goutte, à une température voisine de 0 °C, 26 cm3 d'éthérate de trifluorure de bore. Le mélange est ensuite agité pendant 2 heures à une température voisine de 20 °C puis ajouté à 50 cm3 d'eau distillée. La phase organique est séparée puis lavée successivement par 30 cm3 d' une solution aqueuse 1 N de thiosulfate de sodium, 30 cm3 d'eau distillée, 30 cm3 d'une solution aqueuse saturée de bicarbonate de sodium, séchée sur sulfate de magnésium, filtrée puis concentrée à sec sous pression réduite (2,7 kPa) à 40 °C. L'huile obtenue est purifiée par chromatographie sur 200 g de silice (0,063 - 0,2 mm) contenus dans une colonne de 2,5 cm de diamètre en recueillant des fractions de 25 cm3. Les fractions 12 à 20, éluées par un mélange de cyclohexane et d'acétate d'éthyle (1-1 en volumes), sont concentrées à sec sous pression réduite (2,7 kPa) à 40 °C. On obtient, après recristallisation dans l'acétate d'éthyle, 0,8 g d'(éthoxy-4 phényl)-2 phényl-6 dihydro-5,6 4H-oxazine-1,3 ol-5-(5RS, 6SR) fondant à 124 °C.

L' éthoxy-4 N-cinnamyl benzamide-(E) peut être préparé de la manière suivante : en opérant comme à l'exemple 21 pour la préparation du chloro-3 N-cinnamyl benzamide-(E), mais à partir de 6,6 g de cinnamylamine et de 8,3 g d'acide éthoxy-4 benzoïque, on obtient, après recristallisation dans l'acétate d'éthyle, 8,4 g d'éthoxy-4 N-cinnamyl benzamide-(E) fondant à 132 °C.

EXEMPLE 41

A une solution de 1,8 g de (méthylthio-4 phényl)-2 phényl-6 dihydro-5,6 4H-oxazine-1,3 ol-5-(5RS, 6SR) dans 25 cm3 de dichloro-1,2 éthane maintenue sous atmosphère d'argon, on ajoute à une température voisine de 20 °C, 0,79 g d'isocyanate de phényle. La solution obtenue est chauffée à 80 °C pendant 4 heures puis concentrée à sec sous pression réduite (2,7 kPa). On obtient, après recristallisation dans l'acétonitrile, 1 g de (méthylthio-4 phényl)-2 phényl-6 phénylcarbamoyloxy-5 dihydro-5,6 4H-oxazine-1,3-(5RS, 6SR) fondant à 170 °C.

Le (méthylthio-4 phényl)-2 phényl-6 dihydro-5,6 4H-oxazine-1,3 ol-5-(5RS, 6SR) peut être préparé de la manière suivante : à une solution de 4,2 g de N-(époxy-2.3 phényl-3 propyl) méthylthio-4 benzamide-(2RS, 3RS) dans 50 cm3 de dichlorométhane maintenue sous atmosphère d'argon, on ajoute goutte à goutte, à une température voisine de 0 °C, 1,9 cm3 d'éthérate de trifluorure de bore dissous dans 5 cm3 de dichlorométhane. La solution obtenue est ensuite agitée pendant 3 heures à une temperature voisine de 20 °C puis additionnée de 15 cm3 d'une solution aqueuse 1 N de soude en maintenant l'agitation pendant 30 minutes. La phase aqueuse est séparée puis réextraite par 2 fois 50 cm3 de dichlorométhane. Les phases organiques sont réunies, séchées sur sulfate de magnésium, filtrées puis concentrées à sec sous pression réduite (2,7 kPa) à 40 °C. On obtient, après recristallisation dans l'acétonitrile, 1,8 g de (méthylthio-4 phényl)-2 phényl-6 dihydro-5,6 4H-oxazine-1,3 ol-5-(5RS, 6SR) fondant à 130 °C.

Le N-(époxy-2,3 phényl-3 propyl) méthylthio-4 benzamide-(2RS, 3RS) peut être préparé de la manière suivante :

a- Préparation de la solution A :

A une solution de 10 g d'acide méthylthio-4 benzoïque dans 150 cm3 de dichlorométhane maintenue sous atmosphère d'argon, on ajoute par petites portions à une temperature voisine de 0 °C, 9,7 g de carbonyldiimidazole. La solution obtenue (solution A) est ensuite agitée pendant 2 heures à une température voisine de 20 °C pendant que l'on prépare la solution B.

b- Préparation de la solution B :

9,8 g de N-(époxy-2,3 phényl-3 propyl) trifluoroacétamide-(2RS, 3RS) sont ajoutés à 13 cm3 d'une solution aqueuse 3,1 N de potasse. Après 7 minutes d'agitation à une température voisine de 20 °C, la solution obtenue est saturée avec du chlorure de sodium puis extraite par 2 fois 100 cm3 de dichlorométhane. Les phases organiques sont réunies, séchées sur sulfate de magnésium puis filtrées, constituant ainsi une solution chlorométhylénique d'époxy-2,3 phényl-3 propanamine (2RS, 3RS) (solution B).

La solution B est ajoutée goutte à goutte à la solution A. La solution obtenue est ensuite agitée pendant 3 heures à une température voisine de 20 ° C puis versée dans 100 cm3 d'eau distillée. La phase aqueuse est séparée puis réextraite par 2 fois 50 cm3 de dichlorométhane. Les phases organiques sont réunies, lavées par une solution aqueuse saturée en chlorure de sodium, séchées sur sulfate de magnésium, filtrées puis concentrées à sec sous pression réduite (2,7 kPa) à 40 ° C. On obtient, après recristallisation dans l'acétate d'éthyle, 4,2 g de N-(époxy-2,3 phényl-3 propyl) méthylthio-4 benzamide-(2RS, 3RS) fondant à 110 ° C.

EXEMPLE 42

A une solution de 2 g d'(acétyl-4 phényl)-2 phényl-6 dihydro-5,6 4H-oxazine-1,3 ol-5-(5RS, 6SR) dans 25 cm3 de tétrahydrofuranne maintenue sous atmosphère d'argon, on ajoute à une température voisine de 20 ° C, 0,95 g d'isocyanate de phényle. La solution obtenue est chauffée à 50 ° C pendant 5 heures puis concentrée à sec sous pression réduite (2,7 kPa). On obtient, après recristallisation dans l'éthanol, 1,9 g d'-(acétyl-4 phényl)-2 phényl-6 phénylcarbamoyloxy-5 dihydro-5,6 4H-oxazine-1,3-(5RS, 6SR) fondant à 168 ° C.

L' (acétyl-4 phényl)-2 phényl-6 dihydro-5,6 4H-oxazine-1,3 ol-5-(5RS, 6SR) peut être préparé de la manière suivante : à une solution de 6,6 g d'acétyl-4 N-cinnamyl benzamide-(E) dans 60 cm3 de dichlorométhane maintenue sous atmosphère d'argon, on ajoute goutte à goutte, à une température voisine de 0 ° C, 43 cm3 d'une solution 0,55 M d'acide chloro-3 perbenzoïque dans le dichlorométhane. La suspension obtenue est agitée à une température voisine de 0 ° C pendant 5 heures puis filtrée. Au filtrat obtenu maintenu sous atmosphère d'argon, on ajoute goutte à goutte, à une température voisine de 0 ° C, 3 cm3 d'éthérate de trifluorure de bore. Le mélange obtenu est ensuite agité pendant 2 heures à une température voisine de 20 ° C puis ajouté à 100 cm3 d'eau distillée. La phase organique est séparée puis lavée successivement par 30 cm3 d' une solution aqueuse 1 N de thiosulfate de sodium, 30 cm3 d'eau distillée, 30 cm3 d' une solution aqueuse saturée de bicarbonate de sodium, séchée sur sulfate de magnésium, filtrée puis concentrée à sec sous pression réduite (2,7 kPa) à 40 ° C. L'huile obtenue est purifiée par chromatographie sur 200 g de silice (0,063 - 0,2 mm) contenus dans une colonne de 2,5 cm de diamètre en recueillant des fractions de 30 cm3. Les fractions 45 à 63, éluées par un mélange de cyclohexane et d'acétate d'éthyle (1-1 en volumes), sont concentrées à sec sous pression réduite (2,7 kPa) à 40 ° C. On obtient, après recristallisation dans l'acétate d'éthyle, 2 g d'(acétyl-4 phényl)-2 phényl-6 dihydro-5,6 4H-oxazine-1,3 ol-5-(5RS, 6SR) fondant à 130 ° C.

L' acétyl-4 N-cinnamyl benzamide-(E) peut être préparé de la manière suivante : en opérant comme à l'exemple 21 pour la préparation du chloro-3 N-cinnamyl benzamide-(E), mais à partir de 6,65 g de cinnamylamine et de 9,02 g d'acide acétyl-4 benzoïque, on obtient ainsi, après recristallisation dans l'acétate d'éthyle, 6,6 g d'acétyl-4 N-cinnamyl benzamide-(E) fondant à 120 ° C.

EXEMPLE 43

A une solution de 6 g d'(isopropyl-4 phényl)-2 phényl-6 dihydro-5,6 4H-oxazine-1,3 ol-5-(5RS, 6SR) dans 71 cm3 de dichloro-1,2 éthane maintenue sous atmosphère d'argon, on ajoute à une température voisine de 20 ° C, 2,38 g d'isocyanate de phényle. La solution obtenue est chauffée à 80 ° C pendant 3 heures puis concentrée à sec sous pression réduite (2,7 kPa). Le solide obtenu est purifié par chromatographie sur 300 g de silice (0,063 - 0,2 mm) contenus dans une colonne de 3,5 cm de diamètre en recueillant des fractions de 40 cm3. Les fractions 22 à 27, éluées par un mélange de cyclohexane et d'acétate d'éthyle (8-2 en volumes), sont concentrées à sec sous pression réduite (2,7 kPa) à 40 ° C. On obtient, après recristallisation dans un mélange d'éther de pétrole et d'éthanol (7-2 en volumes), 1,6 g d'(isopropyl-4 phényl)-2 phényl-6 phénylcarbamoyloxy-5 dihydro-5,6 4H-oxazine-1,3-(5RS, 6SR) fondant à 144 ° C.

L'(isopropyl-4 phényl)-2 phényl-6 dihydro-5,6 4H-oxazine-1,3 ol-5-(5RS, 6SR) peut être préparé de la manière suivante: à une solution de 5,5 g d'isopropyl-4 N-cinnamyl benzamide-(E) dans 50 cm3 de dichlorométhane maintenue sous atmosphère d'argon, on ajoute goutte à goutte, à une température voisine de 0 ° C, 56,4 cm3 d'une solution 0,39 M d'acide chloro-3 perbenzoïque dans le dichlorométhane. La suspension obtenue est agitée à une température voisine de 20 ° C pendant 2 heures puis filtrée. Au filtrat obtenu et maintenu sous atmosphère d'argon, on ajoute goutte à goutte, à une température voisine de 0 ° C,

2,4 cm3 d'éthérate de trifluorure de bore. Le mélange obtenu est ensuite agité pendant 2 heures à une température voisine de 20 °C puis ajouté à 50 cm3 d'eau distillée. La phase organique est séparée puis lavée successivement par 30 cm3 d' une solution aqueuse 1 N de thiosulfate de sodium, 30 cm3 d'eau distillée, 30 cm3 d' une solution aqueuse saturée de bicarbonate de sodium, séchée sur sulfate de magnésium, filtrée puis concentrée à sec sous pression réduite (2,7 kPa) à 40 °C. L'huile ainsi obtenue est utilisée sans autre purification.

L' isopropyl-4 N-cinnamyl benzamide-(E) peut être préparé en opérant de la manière suivante : en opérant comme à l'exemple 21 pour la préparation du chloro-3 N-cinnamyl benzamide-(E) mais à partir de 6,65 g de cinnamylamine et de 9,02 g d'acide isopropyl-4 benzoïque, on obtient, après recristallisation dans l'acétate d'éthyle, 5,5 g d'isopropyl-4 N-cinnamyl benzamide-(E) fondant à 142 °C.

EXEMPLE 44

En opérant d'une manière analogue à celle décrite à l'exemple 38, mais à partir de 1,1 g de (morpholino-4 phényl)-2 phényl-6 dihydro-5,6 4H-oxazine-1,3 ol-5 (5RS, 6SR) et de 0,46 g d'isocyamate de phényle et après purification du produit brut obtenu par chromatographie sur 60 g de silice (0,063 - 0,2 mm) contenus dans une colonne de 2,5 cm de diamètre, en éluant par fractions de 20 cm3 avec un mélange acétate d'éthyle-méthanol (95-5 en volumes), récupération et concentration sous pression réduite (2,7 KPa), des fractions 4 à 8 puis recristallisation du résidu ainsi obtenu dans un mélange acétate d'éthyle-oxyde d'isopropyle (50-50 en volumes), on obtient 0,55 g de (morpholino-4 phényl)-2 phényl-6 phénylcarbamoyloxy-5 dihydro-5,6 4H-oxazine-1,3-(5RS, 6SR) fondant à 175 °C.

Le (morpholino-4 phényl)-2 phényl-6 dihydro-5,6 4H-oxazine-1,3 ol-5- (5RS, 6SR) peut être préparé de la manière suivante : en opérant comme à l'exemple 38 pour la préparation de (diméthylamino-4 phényl)-2 phényl-6 dihydro-5,6 4H-oxazine-1,3 ol-5-(5RS, 6SR), mais à partir de 1,6 g de N-(époxy-2,3 phényl-3 propyl) morpholino-4 benzamide-(2RS, 3RS) et de 0,65 cm3 d'éthérate de trifluorure de bore dans 11 cm3 de dichlorométhane, et après purification du produit brut par chromatographie sur 80 g de silice (0,063 - 0,2 mm) contenu dans une colonne de 2,5 cm de diamètre, en éluant par fraction de 20 cm3 avec de l'acétate d'éthyle, récupération et concentration à sec sous pression réduite (2,7 KPa) des fractions 2 à 41, on obtient 1,1 g de (morpholino-4 phényl)-2 phényl-6 dihydro-5,6 4H-oxazine-1,3 ol-5-(5RS, 6SR) utilisé tel quel dans les synthèses ultérieures.

Le N-(époxy-2,3 phényl-3 propyl) morpholino-4 benzamide-(2RS, 3RS) peut être préparé de la manière suivante : en opérant comme à l'exemple 38 pour 1- préparation du N-(époxy-2,3 phényl-3 propyl) diméthylamino-4 benzamide-(2RS, 3RS), mais à partir de 3,44 d'acide morpholino-4 benzoïque, 2,7 g de carbonyldiimidazole, 2,72 g de N-(époxy-2,3 phényl-3 propyl) trifluoroacétamide-(2RS, 3RS), 3,2 cm3 d'une solution aqueuse 3,5 N de potasse et de 100 cm3 de dichlorométhane et après recristallisation dans l'acétate d'éthyle, on obtient 1,9 g de N-(époxy-2,3 phényl-3 propyl) morpholino-4 benzamide-(2RS, 3RS) fondant à 150 °C.

L'acide morpholino-4 benzoïque peut être préparé de la manière suivante : on chauffe à une température de 180 °C pendant 20 heures un mélange de 21 g d'acide fluoro-4 benzoïque et de 65 cm3 de morpholine. Après refroidissement on ajoute 100 cm3 d'eau au mélange réactionnel et le pH de la suspension obtenue est ajusté à 6 par addition d'une solution aqueuse 5N d'acide chlorhydrique. Le produit insoluble est séparé par filtration puis remis en suspension dans 100 cm3 d'eau distillée et le pH de la suspension obtenue est ajusté à 11 par addition de lessive de soude.

La phase aqueuse est extraite 2 fois avec 200 cm3 de dichlorométhane, puis acidifiée à pH6 par addition d'une solution aqueuse 5N d'acide chlorhydrique et le produit insoluble est séparé par filtration. Après lavage à l'eau et séchage on obtient 3,5 g d'acide morpholino-4 benzoïque.

EXEMPLE 45

Un mélange de 1,8 g de phényl-6 (trifluoroacétamido-4 phényl)-2 dihydro-5,6 4H-oxazine-1,3 ol-5-(5RS, 6SR) et de 0,7 g d'isocyanate de phényle dans 75 cm3 de dichloro-1,2 éthane est chauffé à reflux pendant 6 heures. Après refroidissement, le mélange réactionnel est concentré à sec sous pression réduite (2,7 KPa) et le résidu obtenu est purifié par chromotographie sur 50 g de silice (0,063 - 0,2 mm) contenus dans une colonne de 2,5 cm de diamètre en éluant par fractions de 20 cm3 avec on mélange acétate d'éthyle-cyclohexane (50-50 en volumes). Les fractions 3 à 7 sont réunies et concentrées à sec sous pression réduite (2,7 KPa). Le résidu obtenu est agité avec 10 cm3 doxyde de diisopropyle et le produit insoluble est

séparé par filtration et séché à l'air. On obtient ainsi 1 g de phényl-6 phénylcarbamoyloxy-5 (trifloroacétamido-4 phényl)-2 dihydro-5,6 4H-oxazine-1,3-(5RS, 6SR) fondant à 210 °C.

Un mélange de 1g de phényl-6 phénylcarbamoyloxy-5 (trifluoroacétamido-4 phényl)-2 dihydro-5,6 4H-oxazine-1,3-(5RS, 6SR) et de 0,3 g de potasse en pastilles dans 25 cm3 d'eau distillée est chauffé à une température voisine de 80 °C pendant 4 heures. Après refroidissement le mélange réactionnel est extrait 2 fois avec 50 cm3 de dichlorométhane et la solution organique est séchée et concentrée à sec sous pression réduite (2,7 kPa). Le résidu obtenu est purifié par chromatographie sur 20 g de silice contenus dans une colonne de 1 cm de diamètre et en éluant par fractions de 10 cm3 avec un mélange acétate d'éthyle-cyclohexane (50-50 en volumes). Les fractions 5 à 12 sont réunies, concentrées à sec et le résidu est séché sous pression réduite (0,1 kPa). On obtient ainsi 0,45 g d'(amino-4 phényl)-2 phényl-6 phénylcarbamoyloxy-5 dihydro-5,6 4H-oxazine-1,3-(5RS, 6SR) fondant à 110 °C.

Le phényl-6 (trifluoroacétamido-4 phényl)-2 dihydro-5,6 4H-oxazine-1,3 ol-5-(5RS, 6SR) peut être préparé de la manière suivante : en opérant comme à l'exemple 38 pour la préparation du (diméthylamino-4 phényl) 2 phényl-6 dihydro-5,6 4H-oxazine-1,3 ol-5-(5RS, 6SR), mais à partir de 3,6 g de N-(époxy-2,3 phényl-3 propyl) trifluoroacétamido-4 benzamide-(2RS, 3RS) et de 1,35 cm3 d'éthérate de trifluorure de bore dans 75 cm 3 de dichlométhane. Après traitement du résidu obtenu par agitation avec 20 cm3 d'oxyde de diisopropyle, filtration et séchage à l'air, on obtient 1,8 g de (trifluoroacétamido-4 phényl)-2 phényl-6 dihydro-5,6 4H-oxazine-1,3 ol-5-(5RS, 6SR) fondant à 210 °C.

Le N-(époxy-2,3 phényl-3 propyl) trifluoroacétamido-4 benzamide-(2RS, 3RS) peut être préparé de la manière suivante : en opérant comme à l'exemple 38 pour la préparation N-(époxy-2,3 phényl-3 propyl) diméthylamino-4 benzamide-(2RS, 3RS) mais à partir de 19,2 g d'acide trifluoroacétamido-4 benzoïque, 13,4 g de carbonydiimidazole, 18 g de N-(époxy-2,3 phényl-3 propyl) trifluoroacétamide, 25 cm3 d'une solution aqueuse de potasse 3N et de 450 cm3 de dichlorométhane, et après purification par chromatographie du résidu obtenu sur 500 g de silice (0,063 - 0,2 mm) contenus dans une colonne de 4,5 cm de diamètre en éluant par fraction de 25 cm3 avec un mélange acétate d'éthyle-cyclohexane (70-30 en volumes), récupération et concentration sous pression réduite (2,7 kPa) des fractions 6 à 14 et recristallisation dans l'oxyde de diisopropyle, on obtient 3,7 g de N-(époxy-2,3 phényl-3 propyl) trifluoroacétamido-4 benzamide-(2RS, 3SR) fondant à 190 °.

L'acide trifluoroacétamido-4 benzoïque peut être préparé selon la méthode décrite par F. WGYGAND et E. LEISING, Chem. Ber., 87, 248 (1954).

## EXEMPLE 46

A une solution de 3,4 g de (pipéridino-4 phényl)-2 phényl-6 dihydro-5,6 4H-oxazine-1,3 ol-5-(5RS, 6SR) dans 35 cm3 de tétrahydrofuranne maintenue sous atmosphère d'argon, on ajoute à une température voisine de 20 °C, 2 g d'isocyanate de phényle. La solution obtenue est chauffée à 50 °C pendant 5 heures puis concentrée à sec sous pression réduite (2,7 kPa). Le solide obtenu est purifié par chromatographie sur 200 g de silice (0,063 - 0,2 mm) contenus dans une colonne de 2,5 cm de diamètre en recueillant des fractions de 30 cm3. Les fractions 9 à 13, éluées par un mélange de cyclohexane et d'acétate d'éthyle (6-4 en volumes), sont concentrées à sec sous pression réduite (2,7 kpa) à 40 °C. Le solide obtenu est à nouveau purifié par chromatographie sur 100 g de silice (0,063 - 0,2 mm) contenus dans une colonne de 2,5 cm de diamètre en recueillant des fractions de 15 cm3. Les fractions 11 à 15, éluées par un mélange de chloroforme et de méthanol (95-5 en volumes) ,sont concentrées à sec sous pression réduite (2,7 kPa) à 40 °C. On obtient, après recristallisation dans un mélange d'éther de pétrole et d"éthanol (9-1 en volumes) 1,5 g de (pipéridino-4 phényl)-2 phényl-6 phénylcarbamoyloxy-5 dihydro-5,6 4H-oxazine-1,3-(5RS, 6SR) fondant à 166 °C.

Le (pipéridino-4 phényl)-2 phényl-6 dihydro-5,6 4H-oxazine-1,3 ol-5-(5RS, 6SR) peut être préparé de la manière suivante : à une solution de 7,6 g de N-(époxy-2,3 phényl-3 propyl) pipéridino-4 benzamide-(2RS, 3RS) dans 100 cm3 de dichlorométhane maintenue sous atmosphère d'argon, on ajoute goutte à goutte, à une température voisine de 0 °C, 4,4 cm3 d'éthérate de trifluorure de bore dissous dans 10 cm3 de dichlorométhane. La solution obtenue est ensuite agitée pendant 30 minutes à une température voisine de 20 °C puis additionnée de 40 cm3 d'une solution aqueuse 1 N de soude en maintenant l'agitation pendant 30 minutes. La phase aqueuse est est séparée puis réextraite par 2 fois 50 cm3 d'acétate d'éthyle. Les phases organiques sont réunies, séchées sur sulfate de magnésium , filtrées puis concentrées à sec sous pression réduite (2,7 kPa) à 40 °C. On obtient, après recristallisation dans un mélange de cyclohexane et d'acétate d'éthyle (1-1 en volumes), 3,4 g de (pipéridino-4 phényl)-2 phényl-6 dihydro-5,6 4H-oxazine-1,3 ol-5-(5RS, 6SR) fondant à 130 °C.

Le N-(époxy-2,3 phényl-3 propyl) pipéridino-4 benzamide-(2RS, 3RS) peut être préparé de la manière suivante :

a- Préparation de la solution A :

A une solution de 11,5 g d'acide pipéridino-4 benzoïque dans 300 cm3 de dichlorométhane maintenue sous atmosphère d'argon, on ajoute par petites portions à une température voisine de 0° C, 9 g de carbonyldiimidazole. La solution obtenue (solution A) est ensuite agitée pendant 2 heures à une température voisine de 20° C pendant que l'on prépare la solution B.

b- Préparation de la solution B :

9 g de N-(époxy-2,3 phényl-3 propyl) trifluoroacétamide-(2RS, 3RS) sont ajoutés à 12,8 cm3 d'une solution aqueuse 3 N de potasse. Après 7 minutes d'agitation à une température voisine de 20° C, la solution obtenue est saturée avec du chlorure de sodium puis extraite par 2 fois 100 cm3 de dichlorométhane. Les phases organiques sont réunies, séchées sur sulfate de magnésium puis filtrées, constituant ainsi une solution chlorométhylénique d'époxy-2,3 phényl-3 propanamine (2RS, 3RS) (solution B).

La solution B est ajoutée goutte à goutte à la solution A. La solution obtenue est ensuite agitée pendant 3 heures à une température voisine de 20° C puis versée dans 100 cm3 d'eau distillée. La phase aqueuse est séparée puis réextraite par 2 fois 50 cm3 de dichlorométhane. Les phases organiques sont réunies, lavées par une solution aqueuse saturée en chlorure de sodium, séchées sur sulfate de magnésium, filtrées puis concentrées à sec sous pression réduite (2,7 kPa) à 40° C. On obtient, après recristallisation dans un mélange d'acétate d'éthyle et d'oxyde de diisoprppyle (1-1 en volumes), 7,6 g de N-(époxy-2,3 phényl-3 propyl) piperidino-4 benzamide-(2RS, 3RS).

L'acide pipéridino-4 benzoïque peut être préparé de la manière suivante : un mélange de 21 g d'acide fluoro-4 benzoïque et de 75 cm3 de pipéridine est chauffé sous agitation dans un autoclave pendant 17 heures à une température de 180° C, puis refroidi jusqu'à une température voisine de 20° C et versé dans 750 cm3 d'eau distillée. Après acidification jusqu'à pH 6-7 par une solution aqueuse 5N d'acide chlorhydrique, le solide formé est séparé par filtration. On obtient, après recristallisation dans l'acétonitrile 11,5 g d'acide pipéridino-4 benzoïque fondant à 230° C.

EXEMPLE 47

On chauffe à reflux pendant 18 heures un mélange de 1,54 g de (N-méthyl trifluoroacétamido-4 phényl)-2 phényl-6 dihydro-5,6 4H-oxazine-1,3 ol-5-(5RS, 6SR) et de 0,6 g d'isocyanate de phényle dans 25 cm3 de dichloro-1,2 éthane. Le mélange réactionnel est ensuite concentré à sec sous pression réduite (2,7 KPa). Après recristallisation du résidu ainsi obtenu dans l'oxyde d'isopropyle, on obtient 0,77 g de (N-méthyl trifluoroacétamido-4 phényl)-2 phényl-6 phénylcarbamoyloxy-5 dihydro-5,6 4H-oxazine-1,3-(5RS, 6SR) fondant à 160° C.

Un mélange de 0,57 g de (N-méthyl trifluoroacétamido-4 phényl)-2 phényl-6 phénylcarbamoyloxy-5 dihydro 5,6 4H-oxazine-1,3-(5RS, 6SR) et de 0,15 g de potasse en pastilles dans 9 cm3 d'eau chauffé à reflux pendant 18 heures. Après refroidissement le mélange réactionnel est extrait 2 fois avec 20 cm3 de dichlorométhane puis la solution organique est séchée et concentrée à sec sous pression réduite (2,7 Kpa). Le résidu obtenu est purifié par chromatographie sur 30 g de silice (0,063-0,2 mm) contenus dans une colonne de 2,5 cm de diamètre en éluant par fractions de 20 cm3 avec un mélange acétate d'éthyle-cyclohexane (30-70 en volumes). Les fractions 7 à 20 sont réunies et concentrées à sec sous pression réduite (2,7 KPa). On obtient ainsi 0,18 g de (méthylamino-4 phényl)-2 phényl-6 phénylcarbamoyloxy-5 dihydro-5,6 4H-oxazine-1,3-(5RS-6SR) fondant à 170° C.

Le (N-méthyl trifluoroacétamido-4 phényl)-2 phényl-6 dihydro-5,6 4H-oxazine-1,3 ol-5-(5RS, 6SR) peut être préparé de la manière suivante : en opérant comme à l'exemple 38, pour la prépa ration du (diméthylamino-4 phényl)-2 phényl-6 dihydro-5,6 4H-oxazine-1,3 ol-5-(5RS, 6SR), mais à partir de 2,13 g de N-(époxy-2,3 phényl-3 propyl) (N-méthyl trifluoroacétamido)-4 benzamide-(2RS, 3RS) et de 0,75 cm3 d'éthérate de trifluorure de bore dans 15 cm3 de dichlorométhane et après chromatographie du produit obtenu sur 150 g de silice (0,063 - 0,2 mm) contenus dans une colonne de 3,5 cm de diamètre, en éluant par fractions de 30 cm3 avec un mélange acétate d'éthyle-cyclohexane (80-20 en volumes), récupération et

concentration sous pression réduite (2,7 KPa) des fractions 11 à 20, on obtient 1,5 g de [(N-méthyltrifluoroacétamido)-4 phényl]-2 phényl-6 dihydro-5,6 4H-oxazine-1,3 ol-5-(5RS, 6SR) sous forme d'une huile utilisée telle quelle dans les phases ultérieures.

Le N-(époxy-2,3 phényl-3 propyl) (N-méthyltrifluoroacétamido)-4 benzamide-(2RS, 3RS) peut être préparé de la manière suivante : en opérant comme à l'exemple 38 pour la préparation du N-(époxy-2,3 phényl-3 propyl) diméthylamino-4 benzamide-(2RS, 3RS), mais à partir de 8 g d'acide (N-méthyltrifluoroacétamido)-4 benzoïque, 5,25 g de carbonyldiimidazole, 5,22 g de N-(époxy-2,3 phényl-3 propyl) trifluoroacétamide, 6 cm3 d'une solution aqueuse 3,5 N de potasse et de 160 cm3 de dichlorométhane et aprés chromatographie du résidu obtenu sur 400 g de silice (0,063 - 0,2 mm) contenus dans une colonne de 4,5 cm de diamètre, en éluant par fractions de 50 cm3 avec un mélange acétate d'éthyle-cyclohexane (80-20 en volumes), récupération et concentration sous pression réduite des fractions 14 à 20, on obtient 2,1 g de N-(époxy-2,3 phényl-3 propyl) (N-méthyltrifluoroacétamido)-4 benzamide-(2RS, 3RS) sous forme d'une huite jaune utilisée telle quelle dans les phases ultérieures.

L'acide (N-méthyl trifluoroacétamido)-4 benzoïque peut être préparé de la façon suivante : à une solution de 21 g d'acide méthylamino-4 benzoïque dans 200 cm3 d'éther diéthylique anhydre, on ajoute une solution de 43,75 g d'anhydride trifluoroacétique dans 50 cm3 d'éther diéthylique anhydre en maintenant la température au voisinage de 0° C. Le mélange réactionnel est ensuite agité pendant 20 heures à une température voisine de 20° C puis il est concentré à sec sous pression réduite (2,7 Kpa). Par recristallisation du résidu obtenu dans un mélange oxyde de diisopropyle-acétate d'éthyle (80-20 en volumes), on obtient 7,5 g d'acide (N-méthyl trifluoroacétamido)-4 benzoïque fondant à 175° C.

## EXEMPLE 48

Un mélange de 6 g de phényl-6 (trifluorométhoxy-4 phényl)-2 dihydro-5,6 4H-oxazine-1,3 ol-5-(5RS, 6RS) et de 2,4 g d'isocyanate de phényle dans 100 cm3 de dichloro-1,2 éthane est chauffé à une température voisine de 60° C pendant 5 heures. Après refroidissement le mélange réactionnel est concentré à sec sous pression réduite (2,7 kPa) et le résidu obtenu est purifié par chromatographie sur 200 g de silice (0,063-0,2 mm) contenus dans une colonne de 3,5 cm de diamètre, en éluant par fractions de 20 cm3 avec un mélange acétate d'éthyle-cyclohexane (30-70 en volumes). Les fractions 4 à 8 sont réunies et concentrées à sec sous pression réduite (2,7 kPa). Le résidu est cristallisé dans 5 cm3 d'oxyde de diisopropyle. On obtient ainsi 1,1 g de phényl-6 phénylcarbamoyloxy-5 (trifluorométhoxy-4 phényl)-2 dihydro-5,6 4H-oxazine-1,3-(5RS, 6SR) fondant à 128° C.

Le phényl-6 (trifluorométhoxy-4 phényl)-2 dihydro-5,6 4H-oxazine-1,3 ol-5-(5RS, 6SR) peut être préparé de la manière suivante : en opérant comme à l'exemple 12 pour la préparation du diphényl-2,6 dihydro-5,6 4H-oxazine-1,3 ol-5-(5RS, 6SR), mais à partir de 13 g de N-cinnamyl trifluorométhoxy-4 benzamide-(E), 95 cm3 d'une solution 0,44 M d'acide métachloroperbenzoïque, 5,4 cm3 d'éthérate de trifluorure de bore et de 150 cm3 de dichlorométhane et aprés chromatographie du produit obtenu sur 200 g de silice (0,063 - 0,2 mm) contenus dans une colonne de 3,5 cm de diamètre en éluant par fractions de 25 cm3 avec un mélange acétate d'éthyle-cyclohexane (40-60 en volumes), récuperation et concentration sous pression réduite (2,7 kPa) des fractions 8 à 14, on obtient 6,4 g de phényl-6 (trifluorométhoxy-4 phényl)-2 dihydro-5,6 4H-oxazine-1,3 ol-5-(5RS, 6SR) fondant à 120"C.

Le N-cinnamyl trifluorométhoxy-4 benzamide-(E) peut être préparé de la manière suivante : en opérant comme à l'exemple 19 pour la préparation du N-cinnamyl méthoxy-4 benzamide-(E) mais à partir de 11,6 g d'acide trifluorométhoxy-4 benzoïque, 5,3 cm3 de dichlorure d'oxalyle, 7,4 g de cinnamylamine-(E), 16 cm3 de triéthylamine, 0,2 cm3 de diméthylformamide, 100 cm3 d'éther diéthylique et de 150 cm3 de dichlorométhane et recristallisation dans l'oxyde de diisopropyle, on obtient 13 g de N-cinnamyl trifluorométhoxy-4 benzamide-(E) fondant à 136° C.

## EXEMPLE 49

Un mélange de 6,5 g d'(éthylthio-4 phényl)-2 phényl-6 dihydro-5,6 4H-oxazine-1,3 ol-5 (5RS, 6SR) et de 2,6 g d'isocyanate de phényle dans 100 cm3 de dichloro-1,2 éthane est chauffé à une température voisine de 80° C pendant 5 heures. Après refroidissement le mélange réactionnel est concentré à sec sous pression réduite (2,7 kPa) et le résidu obtenu est purifié par chromotographie sur 150 g de silice (0,063 - 0,2 mm) en éluant par fractions de 20 cm3 avec un mélange d'acétate d'éthyle et de cyclohexane (50-50 en volumes). Les fractions 5 à 15 sont réunies et concentrées à sec sous pression réduite (2,7 kPa). Le

résidu obtenu est encore purifié par chromatographie sur 100 g de silice (0,063 - 0,2 mm) contenus dans une colonne de 3,5 cm de diamètre, en éluant par fractions de 25 cm3 avec un mélange chlorure de méthylène-méthanol (99-1 en volumes). Les fractions 10 à 18 sont réunies et concentrées à sec sous pression réduite. On obtient ainsi 1,1 d'(éthylthio-4 phényl)-2 phényl-6 phénylcarbamoyloxy-5 dihydro-5,6 4H-oxazine-1,3-(5RS, 6SR) fondant à 70 °C.

L'(éthylthio-4 phényl)-2 phényl-6 dihydro-5,6 4H-oxazine-1,3 ol-5 (5RS, 6SR) peut être préparée de la manière suivante : en opérant comme à l'exemple 38 pour la préparation du (diméthylamino-4 phényl)-2 phényl-6 dihydro-5,6 4H-oxazine-1,3 ol-5-(5RS, 6SR) mais à partir de 10 g de N-(époxy-2,3 phényl-3 propyl) éthylthio-4 benzamide-(2RS, 3RS) et de 4,3 cm3 d'éthérate de trifluorure de bore dans 100 cm3 de dichlorométhane et après cristallisation du résidu obtenu dans un mélange acétate d'éthyle - oxyde de diisopropyle (30-70 en volumes), on obtient 4,2 g d'(éthylthio-4 phényl)-2 phényl-6 dihydro-5,6 4H-oxazine-1,3 ol-5(5RS, 6SR) fondant à 120 °C.

Le N-(époxy-2,3 phényl-3 propyl) éthylthio-4 benzamide peut être préparée de la manière suivante : en opérant comme à l'exemple 38 pour la préparation du N-(époxy-2,3 phényl-3 propyl) diméthylamino benzamide-(2RS, 3RS) mais à partir de 13,6 d'acide éthylthio-4 benzoïque, 12 g de carbonyldiinidazole, 12,25 g de N-(époxy-2,3 phényl-3 propyl) trifluoroacétamide, 16 cm3 d'une solution aqueuse 3,2 N de potasse et 375 cm3 et dichlorométhane et après traitement du résidu obtenu par chromatographie sur 400 g de silice (0,063 - 0,2 mm) contenus dans une colonne de 4,5 cm de diamètre, en éluant par fractions de 25 cm3 avec un mélange acétate d'éthyle cyclohexane (50-50 en volumes), récupération et concentration à sec sous pression réduite (2,7 KPa) des fractions 2 à 5, on obtient 10 g de N-(époxy-2,3 phényl-3 propyl) éthylthio-4 benzamide-(2RS, 3RS) sous forme d'une huile jaune utilisée telle quelle dans les synthèses ultérieures.

EXEMPLE 50

A une solution de 3,2 g de (tert-butyl-4 phényl)-2 phényl-6 dihydro-5,6 4H-oxazine-1,3 ol-5-(5RS, 6SR) dans 35 cm3 de tétrahydrofuranne anhydre, maintenue sous atmosphère d'argon, on ajoute à une température voisine de 20 °C, 2 g d'isocyanate de phényle. Le solution obtenue est chauffée à 50 °C pendant 5 heures puis concentrée à sec sous pression réduite (2,7 kPa). Le solide obtenu est purifié par chromatographie sur 200 g de silice (0,063 -0,2 mm) contenus dans une colonne de 3,5 cm de diamètre en recueillant des fractions de 20 cm3. Les fractions 22 à 29, éluées par un mélange de cyclohexane et d'acétate d'éthyle (7-3 en volumes), sont concentrées à sec sous pression réduite (2,7 kPa) à 40 °C. Le solide obtenu est à nouveau purifié par chromatographie sur 200 g de silice (0,063 - 0,2 mm) contenus dans une colonne de 2,5 cm de diamètre en recueillant des fractions de 10 cm3. Les fractions 19 à 25, éluées par un mélange de dichlorométhane et de méthanol (98-2 en volumes), sont concentrées à sec sous pression réduite (2,7 kPa) à 40 °C. On obtient, après recristallisation dans l'acétate d'éthyle 1,4 g de (tert-butyl-4 phényl)-2 phényl-6 phénylcarbamoyloxy-5 dihydro-5,6 4H-oxazine-1,3-(5RS, 6SR) fondant à 80 °C.

Le (tert-butyl-4 phényl)-2 phényl-6 dihydro-5,6 4H-oxazine-1,3 ol-5-(5RS, 6SR) peut être préparé de la manière suivante: à une solution de 8,9 g de tert-butyl-4 N-cinnamyl benzamide-(E) dans 80 cm3 de dichlorométhane maintenue sous atmosphère d'argon, on ajoute goutte à goutte, à une température voisine de 0 °C, 94,7 cm3 d'une solution 0,38 M d'acide chloro-3 perbenzoïque dans le dichlorométhane. La suspension obtenue est agitée à une température voisine de 20 °C pendant 2 heures puis filtrée. Au filtrat obtenu maintenu sous atmosphère d'argon, on ajoute goutte à goutte, à une température voisine de 0 °C, 3,8 cm3 d'éthérate de trifluorure de bore. Le mélange obtenu est ensuite agité pendant 2 heures à une température voisine de 20 °C puis ajouté à 50 cm3 d'eau distillée. La phase organique est séparée puis lavée successivement par 30 cm3 d' une solution aqueuse 1 N de thiosulfate de sodium, 30 cm3 d'eau distillée, 30 cm3 d' une solution aqueuse saturée de bicarbonate de sodium, séchée sur sulfate de magnésium, filtrée puis concentrée à sec sous pression réduite (2,7 kPa) à 40 °C. L'huile obtenue est purifiée par chromatographie sur 200 g de silice (0,063 - 0,2 mm) contenus dans une colonne de 3,5 cm de diamètre en recueillant des fractions de 20 cm3. Les fractions 50 à 90, éluées par un mélange de cyclohexane et d'acétate d'éthyle (7-3 en volumes), sont concentrées à sec sous pression réduite (2,7 kPa) à 40 °C. On obtient, après recristallisation dans l'acétate d'éthyle, 0,8 g de (tert-butyl-4 phényl)-2 phényl-6 dihydro-5,6 4H-oxazine-1,3 ol-5-(5RS, 6SR) fondant à 50 °C.

Le tert-butyl-4 N-cinnamyl benzamide-(E) peut être préparé de la manière suivante : en opérant d'une manière analogue à celle décrite à l'exemple 21 pour la préparation du chloro-3 N-cinnamyl benzamide-(E) mais à partir de 6,65 g de cinnamylamine et de 9,8 g d'acide tert-butyl-4 benzoïque. On obtient ainsi, après recristallisation dans l'acétate d'éthyle, 8,9 g de tert-butyl-4 N-cinnamyl benzamide-(E).

EXEMPLE 51

A une solution de 2,5 g de (nitro-4 phénoxycarbonyloxy)-5 diphényl-2,6 dihydro-5,6 4H-oxazine-1,3-(5RS, 6SR) dans 30 cm3 de diméthylformamide anhydre maintenue sous atmosphère d'argon, on ajoute à une température voisine de 20 °C, 1 g d'amino-3 phénol. La solution obtenue est chauffée à 70 °C pendant 5 heures puis refroidie jusqu'à one temperature voisine de 20 °C et versée dans 300 cm3 d'eau distillée. Le mélange est extrait par 2 fois 150 cm3 de dichlorométhane. Les phases organiques sont réunies, lavées par 3 fois 20 cm3 d'eau distillée, séchées sur sulfate de magnésium et concentrées à sec sous pression réduite (2,7 kPa) à 40 °C. L'huile obtenue est purifiée par chromatographie sur 100 g de silice (0,063 - 0,2 mm) contenus dans une colonne de 2 cm de diamètre [éluant : cyclohexane-acétate d'éthyle-triéthylamine ( 60-35-5 en volumes)], en recueillant des fractions de 25 cm3. Les fractions 10 à 17 sont réunies et concentrées à sec sous pression réduite (2,7 kPa) à 40 °C. On obtient, après recristallisation dans l'oxyde de diisopropyle, 1,1 g d'(hydroxy-3 phénylcarbamoyloxy)-5 diphényl-2,6 dihydro-5,6 4H-oxazine-1,3-(5RS, 6SR) fondant à 170 °C.

EXEMPLE 52

A 0,27 g d'une suspension huileuse (50% en poids) d'hydrure de sodium dans 15 cm3 de tétrahydrofuranne anhydre maintenue sous atmosphère d'argon, on ajoute à une température voisine de 20 °C, une solution de 1,42 g de (méthoxy-4 phényl)-2 phényl-4 dihydro-5,6 4H-oxazine-1,3 ol-5-(4RS, 5RS) dans 15 cm3 de tétrahydrofuranne anhydre et on agite la solution obtenue pendant 30 minutes à cette même température. On ajoute alors goutte à goutte une solution de 1,05 g d'isocyanate de dichloro-3,4 phényle dans 10 cm3 de tétrahydrofuranne anhydre et l'on poursuit l'agitation pendant 4 heures à une température voisine de 20 °C. On ajoute ensuite 20 cm3 d'eau distillée et on extrait par 3 fois 80 cm3 de dichlorométhane. Les phases organiques sont réunies, lavées par 3 fois 20 cm3 d'eau distillée, séchées sur sulfate de magnésium et concentrées à sec sous pression réduite (2,7 kPa) à 40 °C. Le solide obtenu est purifié par chromatographie sur 300 g de silice (0,063 -0,2 mm) contenus dans une colonne de 3,5 cm de diamètre (éluant : dichlorométhane) en recueillant des fractions de 30 cm3. Les fractions 4 à 7 sont réunies et concentrées à sec sous pression réduite (2,7 kPa) à 40 °C. Le solide obtenu est à nouveau purifié par chromatographie sur 125 g de silice (0,063 - 0,2 mm) contenus dans une colonne de 2 cm de diamètre [éluant : cyclohexane-acétate d'éthyle (6-4 en volumes)] en recueillant des fractions de 20 cm3. Les fractions 5 à 9 sont réunies et concentrées à sec sous pression réduite (2,7 kPa) à 40 °C. On obtient, après recristallisation dans l'oxyde de diisopropyle, 1,1 g de (dichloro-3,4 phénylcarbamoyloxy)-5 (méthoxy-4 phényl)-2 phényl-4 dihydro-5,6 4H-oxazine-1,3-(4RS,5RS) fondant à 130 °C.

EXEMPLE 53

A une solution de 1,8 g de (naphtyl-2)-2 phényl-6 dihydro-5,6 4H-oxazine-1,3 ol-5-(5RS, 6SR) dans 21 cm3 de dichloro-1,2 éthane maintenue sous atmosphère d'argon, on ajoute à une température voisine de 20 °C, 0,7 g d'isocyanate de phényle. La solution obtenue est chauffée à 80 °C pendant 4 heures puis concentrée à sec sous pression réduite (2,7 kPa). Le solide obtenu est purifié par chromatographie sur 200 g de silice (0,063 - 0,2 mm) contenus dans une colonne de 2,5 cm de diamètre en recueillant des fractions de 20 cm3. Les fractions 12 à 20, éluées par du dichlorométhane ,sont concentrées à sec sous pression réduite (2,7 kPa) à 40 °C. On obtient, après recristallisation dans l'acétonitrile 0,9 g de (naphtyl-2)-2 phényl-6 phénylcarbamoyloxy-5 dihydro-5,6 4H-oxazine-1,3-(5RS, 6SR) fondant à 163 °C.

Le (naphtyl-2)-2 phényl-6 dihydro-5,6 4H-oxazine-1,3 ol-5-(5RS, 6SR) peut être préparé de la manière suivante : à une solution de 4,3 g de N-cinnamyl naphtalènecarboxamide-2-(E) dans 40 cm3 de dichlorométhane maintenue sous atmosphère d'argon, on ajoute goutte à goutte, à une température voisine de 0 °C, 49 cm3 d'une solution 0,38 M d'acide chloro-3 perbenzoïque dans le dichlorométhane. La suspension obtenue est agitée à une température voisine de 20 °C pendant 3 heures puis filtrée. Au filtrat obtenu maintenu sous atmosphère d'argon, on ajoute goutte à goutte, à une température voisine de 0 °C, 1,9 cm3 d'éthérate de trifluorure de bore. Le mélange obtenu est ensuite agité pendant 2 heures à une température voisine de 20 °C puis ajouté à 50 cm3 d'eau distillée. La phase organique est séparée puis lavée successivement par 30 cm3 d' une solution aqueuse 1 N de thiosulfate de sodium, 30 cm3 d'eau distil lée, 30 cm3 d' une solution aqueuse saturée de bicarbonate de sodium, séchée sur sulfate de magnésium, filtrée puis concentrée à sec sous pression réduite (2,7 kPa) à 40 °C. On obtient, après recristallisation dans

l'acétonitrile, 1,8 g de (naphtyl-2)-2 phényl-6 dihydro-5,6 4H-oxazine-1,3 ol-5-(5RS, 6SR) fondant à 172 °C.

Le N-cinnamyl naphtalènecarboxamide-2-(E) peut être préparé de la manière suivante : en opérant d'une manière analogue à celle décrite à l'exemple 21 pour la préparation du chloro-3 N-cinnamyl benzamide-(E) mais à partir de 5,3 g de cinnamylamine et de 7,6 g d'acide naphtoïque-2, on obtient, après recristallisation dans l'acétate d'éthyle, 4,3 g de N-cinnamyl naphtalènecarboxamide-2-(E).

La présente invention concerne également les médicaments constitués par au moins un composé de formule (I) à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale, rectale ou topique.

Comme compositions solides pour administration orale, peuvent être utilisés des comprimés, pilules, poudres (capsules de gélatine, cachets) ou granulés. Dans ces compositions, le principe actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent éga- lement contenir des adjuvants, en particulier des agents mouil- lants, isotonisants, émulsifiants, dispersants et stabilisants.

La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les composés selon l'invention sont particulièrement utiles dans le traitement et la prévention des désordres liés à la CCK au niveau du système nerveux et de l'appareil gastrointestinal. Ces composés peuvent donc être utilisés dans le traitement et la prévention des psychoses, de la maladie de Parkinson, de la diskinésie tardive, du syndrome du colon irritable, de la pancréatite aigüe, des ulcères et des désordres de la motilité intestinale, comme potentialisateur de l'activité analgésique des médicaments analgésiques narcotiques et non narcotiques et comme régulateur de l'appétit.

Les doses dépendent de l'effet recherché, de la durée du traitement et de la voie d'administration utilisée ; elles sont généralement comprises entre 0,05 g et 1 g par jour par voie orale pour un adulte avec des doses unitaires allant de 10 mg à 500 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

## EXEMPLE A

On prépare, selon la technique habituelle, des gélules dosées à 50 mg de produit actif ayant la composition suivante :

| | |
|---|---|
| - (chloro-4 phényl)-2 phényl-4 phénylcarbamoyloxy-5 dihydro-5,6 4H-oxazine-1,3-(4RS, 5RS) | 50 mg |
| - cellulose | 18 mg |
| - lactose | 55 mg |
| - silice colloïdale | 1 mg |
| - carboxyméthylamidon sodique | 10 mg |
| - talc | 10 mg |
| - stéarate de magnésium | 1 mg |

EXEMPLE B

On prépare, selon la technique habituelle, des comprimés dosés à 50 mg de produit actif ayant la composition suivante :

| | |
|---|---|
| - (méthylènedioxy-3,4 phényl)-2 phényl-4 phénylcarbamoyloxy-5 dihydro-5,6 4H-oxazine-1,3-(4RS, 5RS) | 50 mg |
| - lactose | 104 mg |
| - cellulose | 40 mg |
| - polyvidone | 10 mg |
| - carboxyméthylamidon sodique | 22 mg |
| - talc | 10 mg |
| - stéarate de magnésium | 2 mg |
| - silice colloïdale | 2 mg |
| mélange d'hydroxyméthylcellulose, glycérine, oxyde de titane (72-3,5-24,5) | q. s. p. 1 comprimé pelliculé terminé à 245 mg |

EXEMPLE C

On prépare une solution injectable contenant 10 mg de produit actif ayant la composition suivante :

| | |
|---|---|
| - (méthoxy-4 phényl)-2 phényl-6 phénylcarbamoyloxy-5 dihydro-5,6 4H-oxazine-1,3-(5RS, 6RS) | 10 mg |
| - acide benzoïque | 80 mg |
| - alcool benzylique | 0,06 cm3 |
| - benzoate de sodium | 80 mg |
| - éthanol à 95 % | 0,4 cm3 |
| - hydroxyde de sodium | 24 mg |
| - propylène glycol | 1,6 cm3 |
| - eau | q. s. p. 4 cm3 |

**Revendications**

1 - Composés de formule :

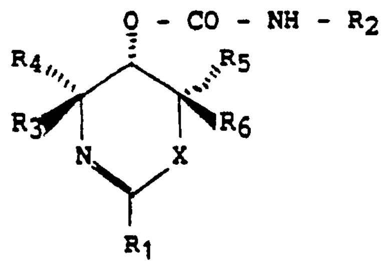

(I)

dans laquelle
- $R_1$ représente un radical indolyl-2, thiényl-2, furyl-3, naphtyle, phényle ou phényle substitué par un ou deux atomes d'halogène, par un radical alcoxy, alkyle, nitro, acylamino, alkylthio, acyle, trifluorométhoxy, morpholino, pipéridino, amino, mono ou dialkylamino dans lequel les groupes alkyle sont identiques ou différents, ou en positions -3 et -4 par un radical méthylènedioxy,
- $R_2$ représente un radical phényle ou phényle substitué par un ou deux atomes d'halogène, par on ou deux radicaux alkyle, par un radical alcoxy, nitro, trifluorométhyle, hydroxy ou en positions -3 et -4 par un radical méthylénedioxy et
- soit X représente un atome d'oxygène et
. $R_3$ représente un radical phényle, $R_4$, $R_5$ et $R_6$ représentent un atome d'hydrogène ou bien
. $R_3$ et $R_4$ représentent un atome d'hydrogène, $R_5$ représente un atome d'hydrogène ou un radical méthyle et $R_6$ représente un radical phényle ou bien
. un des substituants $R_3$ ou $R_4$ représente un radical méthyle et l'autre un atome d'hydrogène, $R_5$ représente un Btome d'hydrogène et $R_6$ représente un radical phényle
- soit X représente un atome de soufre, $R_3$ représente un radical phényle et $R_4$, $R_5$ et $R_6$ représentent un atome d'hydrogène ;
étant entendu que les radicaux acyle, alkyle et alcoxy et les portions acyle, alkyle et alcoxy contiennent 1 à 4 atomes de carbone en chaîne droite ou ramifiée,
ainsi que les sels de ces composés avec un acide minéral ou organique.

2 - Composés de formule (I) selon la revendication 1 pour lesquels $R_1$ représente un radical thiényl-2, naphtyle ou phényle éventuellement substitué par un ou deux atomes d'halogène, par un radical alcoxy, alkyle, nitro, acyle, alkylthio, trifluorométhoxy, morpholino, pipéridino, amino, mono ou dialkylamino dans lequel les groupes alkyle sont identiques ou différents, ou en positions -3 et -4 par un radical méthylène-dioxy, $R_2$ représente un radical phényle éventuellement substitué par un ou deux atomes d'halogène, par un radical alcoxy, nitro, alkyle, hydroxy, trifluorométhyle ou en positions -3 et -4 par un radical méthylène-dioxy, $R_3$, $R_4$, $R_5$ et $R_6$ ont les mêmes significations que dans la revendication 1 étant entendu que les radicaux acyle, alkyle et alcoxy et les portions acyle, alkyle et alcoxy contiennent 1 à 4 atomes de carbone en chaîne droite ou ramifiée, ainsi que les sels de ces composés avec un acide minéral ou organique.

3 - Procédé de préparation des composés de formule (I) selon la revendication 1 à l'exception de ceux pour lesquels $R_2$ représente un radical phényle substitué par un radical hydroxy caractérisé en ce que l'on fait réagir un isocyanate de formule :
$R_2$ - NCO (II)
dans laquelle $R_2$ a les mêmes significations que dans la formule (I), à l'exception de représenter un radical phényle substitué par un radical hydroxy, sur un composé de formule :

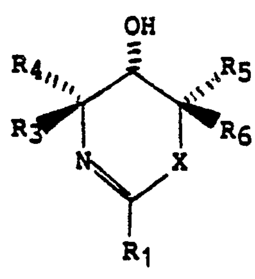

(III)

dans laquelle X, $R_1$, $R_3$, $R_4$, $R_5$ et $R_6$ ont les mêmes significations que dans la revendication 1, ou, lorsque l'un des substituants $R_3$ ou $R_4$ représente un radical méthyle et l'autre un atome d'hydrogène, un mélange de ces composés, puis isole le produit, sépare éventuellement les énantiomères et transforme eventuellement le produit en sel d'addition avec un acide minéral ou organique.

4 - Procédé de préparation des composés de formule (I) selon la revendication 1 caractérisé en ce que l'on fait réagir une amine de formule :

$R_2$ - $NH_2$ (XII)

dans laquelle $R_2$ a les mêmes significations que dans la revendication 1, sur un composé de formule :

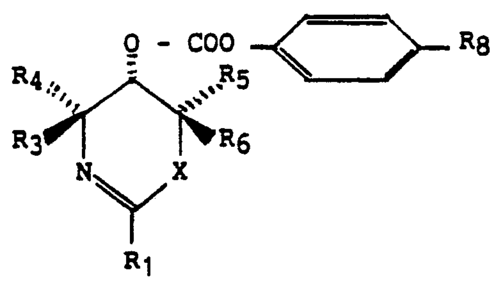

(XIII)

dans laquelle $R_8$ représente un atome d'hydrogène ou un radical nitro et X, $R_1$, $R_3$, $R_4$, $R_5$ et $R_6$ ont les mêmes significations que dans la revendication 1, ou, lorsque l'un des substituants $R_3$ ou $R_4$ représente un radical méthyle et l'autre un atome d'hydrogène, un mélange de ces composés, puis isole le produit, sépare éventuellement les énantiomères et transforme éventuellement le produit en sel d'addition avec un acide minéral ou organique.

5 - Médicaments caractérisés en ce qu'ils contiennent, comme principe actif, au moins un composé selon la revendication 1.

Revendications pour les Etats contractants suivants: GR, ES

1 - Procédé de préparation des composés de formule :

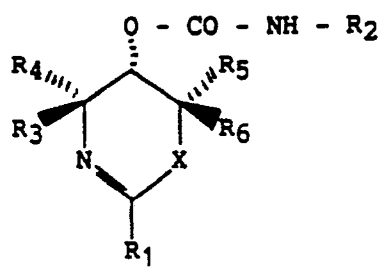

(I)

dans laquelle

- $R_1$ représente un radical indolyl-2, thiényl-2, furyl-3, naphtyle, phényle ou phényle substitué par un ou deux atomes d'halogène, par un radical alcoxy, alkyle, nitro, acylamino, alkylthio, acyle, trifluorométhoxy, morpholino, pipéridino, amino, mono ou dialkylamino dans lequel les groupes alkyle sont identiques ou différents, ou en positions -3 et -4 par un radical méthylènedioxy,
- $R_2$ représente un radical phényle ou phényle substitué par un ou deux atomes d'halogène, par un ou deux radicaux alkyle, par un radical alcoxy, nitro, trifluorométhyle, hydroxy ou en positions -3 et -4 par un radical méthylènedioxy et
- soit X représente un atome d'oxygène et
  . $R_3$ représente un radical phényle, $R_4$, $R_5$ et $R_6$ représentent un atome d'hydrogène ou bien
  . $R_3$ et $R_4$ représentent un atome d'hydrogène, $R_5$ représente un atome d'hydrogène ou un radical méthyle et $R_6$ représente un radical phényle ou bien
  . un des substituants $R_3$ ou $R_4$ représente un radical méthyle et l'autre un atome d'hydrogène, $R_5$ représente un atome d'hydrogène et $R_6$ représente un radical phényle
- soit X représente un atome de soufre, $R_3$ représente un radical phényle et $R_4$, $R_5$ et $R_6$ représentent un atome d'hydrogène ;

étant entendu que les radicaux acyle, alkyle et alcoxy et les portions acyle, alkyle et alcoxy contiennent 1 à 4 atomes de carbone en chaîne droite ou ramifiée,
ainsi que les sels de ces composés avec un acide minéral ou organique,
caractérisé en ce que

A - pour la préparation des composés de formule (I) à l'exception de ceux pour lesquels $R_2$ représente un radical phényle substitué par un radical hydroxy, on fait réagir un isocyanate de formule :
$R_2$ - NCO (II)
dans laquelle $R_2$ a les mêmes significations que précédemment, à l'exception de représenter un radical phényle substitué par un radical hydroxy sur un composé de formule :

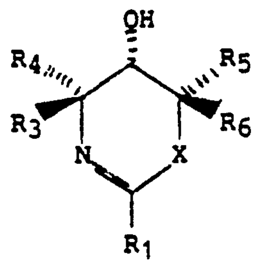

(III)

dans laquelle X, $R_1$, $R_3$, $R_4$, $R_5$ et $R_6$ ont les mêmes significations que ci-dessus, ou, lorsque l'un des substituants $R_3$ ou $R_4$ représente un radical méthyle et l'autre un atome d'hydrogène, un mélange de ces composés puis isole le produit, sépare éventuellement les énantiomères et le transforme éventuellement en sel d'addition avec un acide minéral ou organique.

B - pour la préparation des composés de formule (I), on fait réagir une amine de formule :
$R_2$ - $NH_2$ (XII)
dans laquelle $R_2$ a les mêmes significations que précédemment, sur un composé de formule :

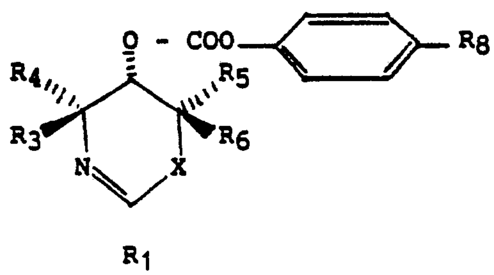

(XIII)

dans laquelle $R_8$ représente un atome d'hydrogène ou un radical nitro et X, $R_1$, $R_3$, $R_4$, $R_5$ et $R_6$ ont les mêmes significations que précédemment ou, lorsque l'un des substituants $R_3$ ou $R_4$ représente un radical méthyle et l'autre un atome d'hydrogène, un mélange de ces composés puis isole le produit, sépare éventuellement les énantiomères et le transforme éventuellement en sel d'addition avec un acide minéral ou organique.

2 - Procédé selon la revendication 1 pour la préparation des composés de formule (I) pour lesquels $R_1$ représente un radical thiényl-2, naphtyle ou phényle éventuellement substitué par un ou deux atomes d'halogène, par un radical alcoxy, alkyle, nitro, acyle, alkylthio, trifluorométhoxy, morpholino, pipéridino, amino, mono ou dialkylamino dans lequel les groupes alkyle sont identiques ou différents, ou en positions -3 et -4 par un radical méthylènedioxy, $R_2$ représente un radical phényle éventuellement substitué par un ou deux atomes d'halogène, par un radical alcoxy, nitro, alkyle, hydroxy, trifluorométhyle ou en positions -3 et -4 par un radical méthylénedioxy, $R_3$, $R_4$, $R_5$ et $R_6$ ont les mêmes significations que dans la revendication 1 étant entendu que les radicaux acyle, alkyle et alcoxy et les protions acyle, alkyle et alcoxy contiennent 1 à 4 atomes de carbone en chaîne droite ou ramifiée, ainsi que les sels de ces composés avec un acide minéral ou organique.

Office européen des brevets

# RAPPORT DE RECHERCHE EUROPEENNE

Numero de la demande

EP 89 40 3029

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 151 279 (M.S. SINGER) * Colonne 2, lignes 15-41; revendications * --- | 1,3 | C 07 D 265/10<br>C 07 D 279/06<br>C 07 D 413/04<br>C 07 D 413/12<br>C 07 D 417/04<br>A 61 K 31/535<br>A 61 K 31/54 |
| A | US-A-2 704 757 (C.A. DORNFELD) * En entier * ----- | 1,5 | |
| | | | **DOMAINES TECHNIQUES RECHERCHES (Int. Cl.5)** |
| | | | C 07 D 265/00<br>C 07 D 279/00<br>C 07 D 413/00<br>C 07 D 417/00<br>A 61 K 31/00 |

Le présent rapport a été établi pour toutes les revendications

| Lieu de la recherche | Date d'achèvement de la recherche | Examinateur |
|---|---|---|
| LA HAYE | 18-01-1990 | CHOULY J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)